# EUROPEAN PATENT APPLICATION

(11) **EP 4 296 660 A1**
(43) Date of publication of application: **27.12.2023**
(21) Application number: 22755933.3
(22) Date of filing: 02.02.2022
(51) Int. Cl.: G01N 27/62

(54) **CALIBRANT FOR USE IN MASS SPECTROMETER AND METHOD FOR PRODUCING SAME**

(30) Priority: 22.02.2021 JP 2021026450
(71) Applicant: Shimadzu Corporation, Kyoto 604-8511 (JP)
(72) Inventor: NISHIKAZE, Takashi, Kyoto-shi, Kyoto 604-8511 (JP); KANEKO, Naoki, Kyoto-shi, Kyoto 604-8511 (JP)
(74) Representative: Mewburn Ellis LLP
(86) International application number: PCT/JP2022/003972
(87) International publication number: WO 2022/176612

(57) **Abstract**

A calibrant for use in correction of machine difference of mass spectrometers and a method for producing the calibrant are provided. A calibrant for use in a mass spectrometer, the calibrant comprising not less than two calibration substances, wherein a ratio of, relative to a concentration of one calibration substance of the not less than two calibration substances, a concentration of another calibration substance of the not less than two calibration substances is a predetermined value, and a method for producing the calibrant.

## Description

### TECHNICAL FIELD

The present invention relates to a calibrant for use in mass spectrometry and a method for producing thereof.

### BACKGROUND ART

When a comparative analysis of abundances of substances is performed using a mass spectrometer, a method using the intensity ratio of two signal peaks is most commonly used. For example, a certain amount of an internal standard substance is added to samples to be compared, the samples are optionally pretreated, and then subjected to mass spectrometry, and a comparison is made between the values of intensity ratio of the peak of an analyte to be measured relative to the peak of the internal standard substance (Non-Patent Documents 1, 2 and 3).

Examples of patent documents include WO 2015/178398 (US 2017/0184573), and WO 2017/047529 (US 2018/0238909).

In addition, a semi-quantitative comparative analysis can be performed by labeling a target substance derived from each of different samples with labeled compounds different in mass due to the use of a stable isotope element and calculating the intensity ratio of peaks of the target substance different in mass by mass spectrometry. This technique includes ICAT (registered trademark) and iTRAQ (registered trademark) used in the field of proteomics (Non-Patent Documents 4 and 5).

### CITATION LIST

### PATENT DOCUMENTS

Patent Document 1: WO 2015/178398
Patent Document 2: US 2017/0184573
Patent Document 3: WO 2017/047529
Patent Document 4: US 2018/0238909

### NON-PATENT DOCUMENTS

Non-Patent Document 1: Kaneko N, Nakamura A, Washimi Y, Kato T, Sakurai T, Arahata Y, Bundo M, Takeda A, Niida S, Ito K, Toba K, Tanaka K, Yanagisawa K. : Novel plasma biomarker surrogating cerebral amyloid deposition. Proc Jpn Acad Ser B Phys Biol Sci. 2014; 90 (9): 353-364.
Non-Patent Document 2: Nakamura A, Kaneko N, Villemagne VL, Kato T, Doecke J, Doré V, Fowler C, Li QX, Martins R, Rowe C, Tomita T, Matsuzaki K, Ishii K, Ishii K, Arahata Y, Iwamoto S, Ito K, Tanaka K, Masters CL, Yanagisawa K. : High performance plasma amyloid-β biomarkers for Alzheimer's disease. Nature. 2018; 554 (7691): 249-254.
Non-Patent Document 3: Nicol GR, Han M, Kim J, Birse CE, Brand E, Nguyen A, Mesri M, FitzHugh W, Kaminker P, Moore PA, Ruben SM, He T : Use of an immunoaffinity-mass spectrometry-based approach for the quantification of protein biomarkers from serum samples of lung cancer patients. Mol Cell Proteomics. 2008 Oct; 7 (10): 1974-82.
Non-Patent Document 4: Han DK, Eng J, Zhou H, Aebersold R : Quantitative profiling of differentiation-induced microsomal proteins using isotope-coded affinity tags and mass spectrometry. Nat Biotechnol. 2001 Oct; 19 (10): 946-51.
Non-Patent Document 5: Ross PL, Huang YN, Marchese JN, Williamson B, Parker K, Hattan S, Khainovski N, Pillai S, Dey S, Daniels S, Purkayastha S, Juhasz P, Martin S, Bartlet-Jones M, He F, Jacobson A, Pappin DJ : Multiplexed protein quantitation in Saccharomyces cerevisiae using amine-reactive isobaric tagging reagents. Mol Cell Proteomics. 2004 Dec; 3 (12): 1154-69.

### SUMMARY OF THE INVENTION

### PROBLEMS TO BE SOLVED BY THE INVENTION

When very small amounts of substances are measured using a mass spectrometer, it is confirmed that a phenomenon occurs in which even when machines of the same type are used, a detected peak intensity ratio varies between the machines. One of means for calibrating such a difference in peak intensity ratio between different machines is the following absolute quantitation method.

A standard product of an analyte substance to be quantitated and a substance used as a reference for measuring an intensity ratio (as a reference substance, a labeled substance of the analyte substance with a stable isotope is generally used) are prepared. Samples containing the reference substance at a certain concentration and the standard product at different concentrations are measured to prepare a calibration curve of the peak intensity ratio of the standard product relative to the reference substance. The absolute quantitation of the analyte substance can be performed using this calibration curve. Therefore, even when there is a difference in peak intensity ratio between different machines, an unknown analyte substance present in a biological sample can be quantitated without the influence of such a difference by preparing a calibration curve for every machine and every measurement.

However, this method using a calibration curve requires a standard product. When a standard product cannot easily be synthesized, or when the kinds of analyte substances are very many and therefore it is difficult to prepare the standard products of all the analyte substances and control quality thereof in terms of time and cost, or when a standard product is unstable, a calibration curve cannot be prepared. As described above, the peak intensity ratio varies between different mass spectrometer machines. Therefore, when a calibration curve cannot be prepared, samples to be compared need to be measured by one machine. However, it is difficult to obtain consistent data because a detector deteriorates due to the use of the machine so that the peak intensity ratio varies.

In mass spectrometry, a sample is ionized by laser irradiation. Therefore, data is influenced by the conditions of a laser of a mass spectrometer (deterioration with, for example, an increase in the number of uses). Therefore, when the same sample is measured by different machines, there is not a little difference in data between the machines. Therefore, the data lacks in stability.

In order to cancel such a difference between mass spectrometer machines, it is considered that correction of measurement data is performed in each of the mass spectrometers.

It is an object of the present invention to provide a calibrant for use in correction of a difference between mass spectrometer machines, and a method for producing the calibrant.

### MEANS FOR SOLVING THE PROBLEMS

The present invention includes the following aspect.

A calibrant for use in a mass spectrometer, the calibrant comprising not less than two calibration substances, wherein
a ratio of, relative to a concentration of one calibration substance of the not less than two calibration substances, a concentration of another calibration substance of the not less than two calibration substances is a predetermined value.

The present invention also includes the following aspect.

A method for producing a calibrant for use in a mass spectrometer, the calibrant comprising not less than two calibration substances, wherein
a ratio of, relative to a concentration of one calibration substance of the not less than two calibration substances, a concentration of another calibration substance of the not less than two calibration substances is a predetermined value, and
the method comprising:
   a previous step of preparing:
      a solution A which comprises one calibration substance (S1) of the not less than two calibration substances at a predetermined concentration, and
      a solution B which comprises the one calibration substance (S1) of the not less than two calibration substances at a concentration equal to the above predetermined concentration, and further comprises another calibration substance (S2) of the not less than two calibration substances; and
   a preparation step of obtaining a calibrant in which a ratio of, relative to a concentration of the one calibration substance (S1), a concentration of the another calibration substance (S2) is a predetermined value by using the solution A and the solution B.

The present invention also includes the following aspect.

A calibrant kit for use in a mass spectrometer, the kit comprising a plurality of calibrants which comprises not less than two calibration substances, wherein
the plurality of calibrants are respectively different in a concentration of at least one calibration substance of the calibration substances.

The present invention also includes the following aspect.

A calibrant kit for use in a mass spectrometer, the kit comprising not less than two calibration substances.

### EFFECTS OF THE INVENTION

According to the present invention, a calibrant for use in correction of a difference between mass spectrometer machines is provided. The calibrant of the present invention can be measured in a mass spectrometer, and, from the measurement results, a calibration formula for the mass spectrometer can be calculated. Measurement results of analyte substances in the mass spectrometer can be calibrated using the calculated calibration formula. Therefore, even when the same sample is measured by different machines respectively, an equivalent peak intensity ratio of the analyte substances can be obtained.

According to the present invention, a method for producing a calibrant for use in correction of a difference between mass spectrometer machines is provided. By using the production method of the present invention, a calibrant containing not less than two calibration substances can be accurately produced in which a ratio of, relative to a concentration of one calibration substance, a concentration of another calibration substance is prepared to a predetermined value. As a result, the calibration of a difference between mass spectrometer machines can be performed with high accuracy.

According to the present invention, a calibrant kit for use in correction of a difference between mass spectrometer machines is provided. Alternatively, a kit for preparing a calibrant is provided. By using the calibrant kit of the present invention, a calibrant containing not less than two calibration substances can be obtained in which a ratio of, relative to a concentration of one calibration substance, a concentration of another calibration substance is prepared to a predetermined value precisely. As a result, the calibration of a difference between mass spectrometer machines can be performed with high accuracy.

### BRIEF DESCRIPTION OF THE DRAWINGS

[Fig. 1] Fig. 1 is a conceptual diagram of one example (a first embodiment) of a preparation step, in the case of producing five calibrants (represented as IC-1, IC-2, IC-3, IC-4 and IC-5).
[Fig. 2] Fig. 2 is a conceptual diagram of another example (a second embodiment) of a preparation step, in the case of producing five calibrants (represented as IC-1, IC-2, IC-3, IC-4 and IC-5).
[Fig. 3] Fig. 3 is a schematic diagram showing a production method of a calibrant according to Example 1.
[Fig. 4] Fig. 4 is a schematic diagram showing a production method of a calibrant according to Example 2.
[Fig. 5] Fig. 5 is a bar chart showing an average value of values b calculated by using calibrants produced by each of the operator 1 and the operator 2 in Example 2. The vertical axis represents the value b. The error bar represents a standard deviation of three values b obtained from three measurement of the calibrants produced by each of the operator 1 and the operator 2.

### MODES FOR CARRYING OUT THE INVENTION

An embodiment of a calibrant for use in a mass spectrometer according to the present invention includes not less than two calibration substances, wherein
a ratio of, relative to a concentration of one calibration substance of the not less than two calibration substances, a concentration of another calibration substance of the not less than two calibration substances is a predetermined value.

This embodiment will be described in detail below.

A calibrant according to this embodiment is used for calibrating a machine difference between mass spectrometer machines. The calibrant according to this embodiment is measured by a mass spectrometer, and a calibration formula for said mass spectrometer is calculated using the measurement results of the calibrant. In said mass spectrometer, a sample containing an analyte substance is measured, and then the measurement data of the sample is calibrated by using a calibration value. The detailed calibration method will be described later.

### [1. Calibration substances]

In this embodiment, a calibration substance is a substance for use in calibration of a machine difference between mass spectrometer machines. A solution (a calibrant) containing not less than two calibration substances is measured by a mass spectrometer. A calibration formula for the mass spectrometer is calculated using the measurement results of the calibration substances.

The calibration substances may appropriately be determined by those skilled in the art. For example, analyte substances per se may be used or substances different from analyte substances may be used. Stable isotope-labeled substances may be used. A substance labeled with a stable isotope and a substance not labeled with a stable isotope may be used. A pair of a substance labeled with a stable isotope and a substance not labeled with a stable isotope may be used.

The calibration substances may be Aβ and an Aβ related peptide(s). The "Aβ and an Aβ related peptide" may simply collectively be called "Aβ related peptides". The "Aβ and an Aβ related peptide" includes Aβ generated by cleaving amyloid precursor protein (APP) and peptides containing even part of the sequence of Aβ.

When analyte substances are Aβ and an Aβ-related peptide(s), for example, stable isotope-labeled Aβ1-38 (SIL-Aβ1-38) may be used as one of the calibration substances. Here, SIL is an abbreviation for stable isotope-labeled. For example, in SIL-Aβ1-38 used in Example, carbon atoms in phenylalanine (Phe or F) and isoleucine (Ile or I) of Aβ1-38 (SEQ ID NO.: 11) are substituted by ¹³C.

The calibration substances may include a compound and said compound labeled with a stable isotope. In mass spectrometry, a compound is ionized for detection. The efficiency of ionization differs depending on the kind of compound, and such a difference in ionization efficiency has an influence on the measurement result of mass spectrometry. A certain compound and said compound labeled with a stable isotope are the same in ionization efficiency. Therefore, a calibration formula with higher accuracy can be obtained using a pair of a compound and said compound labeled with a stable isotope as calibration substances. For example, Aβ1-38 and a stable isotope-labeled Aβ1-38 (SIL-Aβ1-38) may be used as calibration substances. Not limited to Aβ1-38, other substances may be used. For example, other Aβ related peptides, peptides other than Aβ related peptides, proteins, glycans, glycopeptides, glycoproteins, lipids, glycolipids, low molecular medicines or the like may be used. In such a case, these substances may be used as calibration substances in combination of a substance and said substance labeled with a stable isotope.

### [2. Calibrant]

A calibrant is a solution containing a calibration substance. In this embodiment, as a calibrant, a solution containing not less than two calibration substances is used.

As the calibrant, for example, a sample per se containing analyte substances may be used. Alternatively, the calibrant may be one obtained by adding calibration substances to a sample per se containing analyte substances. A solution containing not less than two calibration substances may be prepared and used separately from a sample per se containing analyte substances.

In the calibrant according to this embodiment, a ratio of, relative to a concentration of one calibration substance of the not less than two calibration substances in the calibrant, a concentration of another calibration substance of the not less than two calibration substances in the calibrant is a predetermined value. That is, the ratio of the concentrations in the calibrant is known.

The calibrant according to this embodiment may be produced by adjusting the ratio of the concentrations to a desired value. The ratio of the concentrations may appropriately be determined by those skilled in the art. The ratio of the concentrations may be, for example, in the range of 1/4 to 4. The ratio of the concentrations may be previously confirmed to be a predetermined value by measuring a concentration of each of the calibration substances using a known method.

For example, when the calibrant contains two calibration substances and the calibration substances are represented as S1 and S2, a ratio S2/S1 of, relative to a concentration of one calibration substance (S1), a concentration of the other calibration substance (S2) is a predetermined value in the calibrant.

For example, a substance labeled with a stable isotope may be used as the calibration substance (S1), and a substance not labeled with a stable isotope may be used as the calibration substance (S2). A substance labeled with a stable isotope and said substance not labeled with a stable isotope may be used in combination as the calibration substance (S1) and the calibration substance (S2). For example, a stable isotope-labeled Aβ1-38 (SIL-Aβ1-38) may be used as the calibration substance (S1), and Aβ1-38 not labeled with a stable isotope may be used as the calibration substance (S2). Alternatively, a substance not labeled with a stable isotope may be used as the calibration substance (S1), and a substance labeled with a stable isotope may be used as the calibration substance (S2).

For example, a calibrant may contain not less than three calibration substances. In this case, a ratio of, relative to a concentration of one calibration substance of the not less than three calibration substances, a concentration of any one of other calibration substances of the not less than three calibration substances is a predetermined value in the calibrant.

A case where a calibrant contains three calibration substances is assumed. When the calibration substances are represented as S1, S2, and S3, a ratio S2/S1 of, relative to a concentration of one calibration substance (for example, S1), a concentration of any one of other calibration substances (for example, S2) is a predetermined value in the calibrant. In this case, a ratio S3/S1 of, relative to a concentration of the one calibration substance (S1), a concentration of a calibration substance (in this case, S3) other than S1 and S2 may also be a predetermined value.

A calibration formula for the mass spectrometer is calculated as described later, using the measurement result of the calibrant solution containing not less than two calibration substances. In order to calculate a calibration formula, two or more pieces of data are required. For example, when a calibration formula is calculated using a signal peak intensity ratio, two or more pieces of data different in signal peak intensity ratio are required.

For example, a calibrant may be two or more calibrants which are different in the concentrations of calibration substances whose signal peak intensity ratio should be determined when a calibration formula is calculated. A calibrant may be two or more calibrants that contains two kinds of calibration substances, and are different in a ratio of, relative to a concentration of one of the calibration substances, a concentration of the other calibration substance.

For example, when calibration substances are represented as S1 and S2, a calibrant may be a plurality of calibrants which are different in a ratio S2/S1 of, relative to a concentration of one calibration substance (S1), a concentration of the other calibration substance (S2). The concentration ratios S2/S1 may be, for example, in the range of 1/4 to 4. For example, the calibrant may be composed of five kinds of calibrant solutions whose concentration ratios S2/S1 are adjusted to 1/4, 1/2, 1, 2, and 4, respectively.

For example, a substance labeled with a stable isotope may be used as the calibration substance (S1), and a substance not labeled with a stable isotope may be used as the calibration substance (S2). A substance labeled with a stable isotope and said substance not labeled with a stable isotope may be used in combination as the calibration substance (S1) and the calibration substance (S2). For example, a stable isotope-labeled Aβ1-38 (SIL-Aβ1-38) may be used as the calibration substance (S1), and Aβ1-38 may be used as the calibration substance (S2).

When such a plurality of calibrants is used, the plurality of the calibrants may be used in [5-3-2. Calibration by standardizing signal peak intensity ratio (intensity ratio calibration)] that will be described later. As described later, a ratio of, relative to a signal peak intensity of one calibration substances, a signal peak intensity of the other calibration substance may be calculated for each of the calibrants to obtain not less than two signal peak intensity ratios.

Alternatively, a calibrant may be two or more calibrants that contains not less than three kinds of calibration substances, and are different in a ratio of, relative to a concentration of one of the calibration substances, a concentration of any one of other calibration substances. For example, when the calibrant contains three kinds of calibration substances and the calibration substances are represented as S1, S2, and S3, the calibrant may be a plurality of calibrants which are different in a ratio S2/S1 of, relative to a concentration of one calibration substance (for example, S1), a concentration of any one of other calibration substances (for example, S2).

Alternatively, in the two or more calibrants which are different in the ratios S2/S1, for example, a ratio (a concentration ratio S3/S1) of, relative to a concentration of the calibration substance (S1), a concentration of the calibration substance (S3) may be adjusted to the same or a different predetermined value in each of the calibrants.

It should be noted that, when the calibrant includes not less than three kinds of calibration substances, it is possible to calculate a calibration formula for a mass spectrometer by using one calibrant. The one calibrant may be used, for example, in [5-3-1. Calibration using calibration formula using standard machine] that will be described later.

### [3. Production method of calibrant]

An embodiment of a production method of a calibrant according to the present invention includes:
a previous step of preparing:
a solution A which comprises one calibration substance (S1) of the not less than two calibration substances at a predetermined concentration, and
a solution B which comprises the one calibration substance (S1) of the not less than two calibration substances at a concentration equal to the above predetermined concentration, and further comprises another calibration substance (S2) of the not less than two calibration substances; and
a preparation step of obtaining a calibrant in which a ratio of, relative to a concentration of the one calibration substance (S1), a concentration of the another calibration substance (S2) is a predetermined value by using the solution A and the solution B.

The calibrant is used for calibrating a difference between mass spectrometer machines. In the calibrant produced by the production method according to this embodiment, a ratio of, relative to a concentration of one calibration substance of the not less than two calibration substances, a concentration of another calibration substance of the not less than two calibration substances is prepared to a predetermined value.

The calibration of a difference between mass spectrometer machines using the calibrant produced by the production method according to this embodiment is conducted, for example, by a calibration formula in the mass spectrometer obtained by using a concentration ratio of each of the calibrants and measurement results (for example, a signal peak intensity ratio) of each of the calibrant in the mass spectrometer, wherein the calibrants are respectively different in a ratio of, relative to a concentration of the one calibration substance, a concentration of the another calibration substance. The detail of the calibration method will be described later.

In order to conduct such a calibration with high accuracy, the calibrant needs to be precisely produced. That is, the calibrant needs to be produced so that a ratio of, relative to a concentration of the one calibration substance, a concentration of the another calibration substance is exactly a desired value.

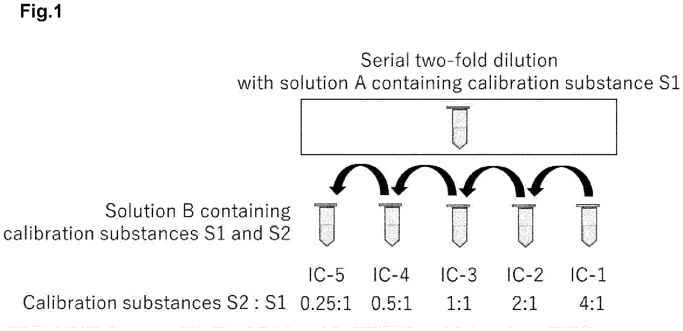

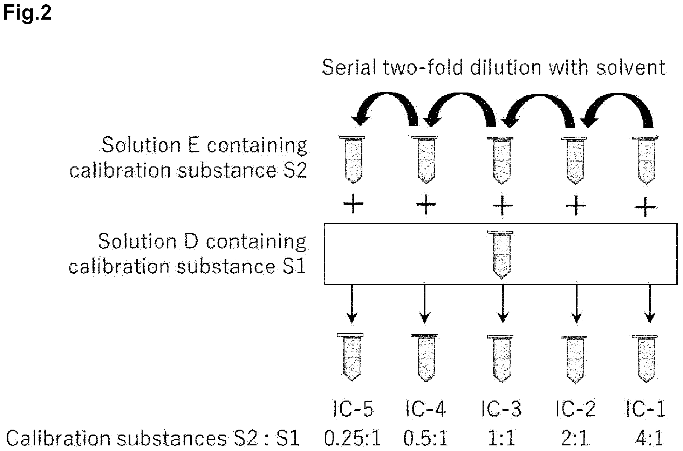

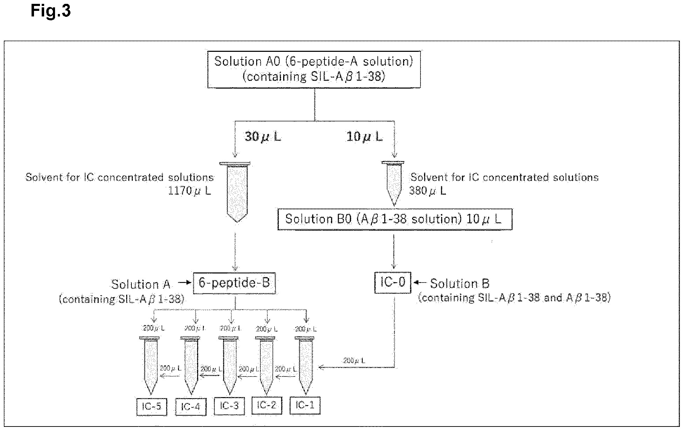

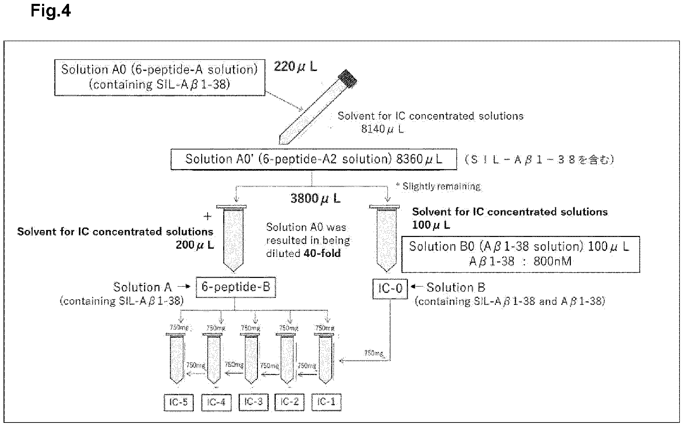

The production method according to this embodiment will be described in detail below. The production method can be explained referring to Figs. 1 to 4. Fig. 1 is a conceptual diagram of a preparation step in a first embodiment. Fig. 2 is a conceptual diagram of a preparation step in a second embodiment. Fig. 3 is a schematic diagram showing a production method of a calibrant according to Example 1. Fig. 4 is a schematic diagram showing a production method of a calibrant according to Example 2.

A calibrant, in which at least two calibration substances (S1 and S2) are included and a ratio (a concentration ratio S2/S1) of a concentration of the calibration substance (S2) in the calibrant relative to a concentration of the calibration substance (S1) in the calibrant is adjusted to a predetermined value, is produced by using the production method according to this embodiment.

A substance labeled with a stable isotope may be used as S1, and a substance not labeled with a stable isotope may be used as S2. Alternatively, a substance not labeled with a stable isotope may be used as S1, and a substance labeled with a stable isotope may be used as S2. A substance labeled with a stable isotope and said substance not labeled with a stable isotope may be used in combination as the calibration substance (S1) and the calibration substance (S2). For example, in Examples, an example of using a stable isotope-labeled Aβ1-38 (SIL-Aβ1-38) as the calibration substance (S1) and Aβ1-38 as the calibration substance (S2) is shown.

### [3-1. Previous step (Pre-process)]

Two solutions are prepared:
a solution A which comprises the calibration substance (S1) at a predetermined concentration, and
a solution B which comprises the calibration substance (S1) at a concentration equal to the above predetermined concentration, and further comprises the calibration substance (S2).

A concentration of the calibration substance (S1) in each of the solution A and the solution B may be the same concentration as a concentration of the calibration substance (S1) in a calibrant to be produced. The concentration of the calibration substance (S1) in each of the solution A and the solution B may be, for example, 1 pmol/L to 1 mmol/L.

A concentration of the calibration substance (S2) in the solution B may be, for example, 0.1 pmol/L to 10 mmol/L. The concentration of the calibration substance (S2) in the solution B may be about 1.1 to 10 times a concentration of the calibration substance (S2) in the calibrant to be produced. The concentration of the calibration substance (S2) in the solution B may be, for example, 1.2 to 5 times that in the calibrant to be produced, and preferably, about 1.5 to 5 times that in the calibrant to be produced. For example, the concentration of the calibration substance (S2) in the solution B may be twice the concentration of the calibration substance (S2) in the calibrant to be produced.

A solvent used for the solution A and the solution B can be appropriately determined by a person skilled in the art. The solvent may be, for example, water, acetonitrile, an alcohol, and the like. The solvent may be used singly or in combination of two or more kinds of them. The solvent used for the solution A and the solvent used for the solution B may be different or the same. Any other substance may be added to the solvent. The solvent may include, for example, bovine serum albumin (BSA), ammonium bicarbonate, and the like. BSA functions as an adsorption preventing agent, and also functions as an antioxidant that inhibits oxidation of methionine, or the like. The solvent may contain, for example, an oxidation inhibitor or the like to maintain quality thereof. Proteins, amino acids, or reducing agents may be used as the oxidation inhibitor. Examples of proteins include BSA, transferrin, and fetuin. Examples of amino acids include methionine, histidine, cysteine, and tryptophan. Examples of reducing agents include dithiothreitol (DTT), tris(2-carboxyethyl)phosphine (TCEP), 2-mercaptoethanol (2-ME), tri-n-butylphosphine (TBP), dithioerythritol (DTE), ascorbic acid, polyphenol, sodium pyrosulfite, citric acid, glucose, carotene, tocopherol, thio-glycolic acid, N-acetylcysteine, hydroxylamine, glutathione (reduced form), 2-aminoethyl isothiouronium bromide, and thioglycerol. The oxidation inhibitor may be used singly or in combination of two or more substances.

For example, the solution A and the solution B may be prepared separately. That is, the solution A may be prepared using the solvent and the calibration substance (S1). And the solution B may be separately prepared using the solvent, the calibration substance (S1), and the calibration substance (S2).

Preferably, a solution A0 containing the calibration substance (S1) at a higher concentration than a desired concentration, and a solution B0 containing the calibration substance (S2) are firstly prepared.

A solvent used for the solution A0 can be appropriately determined by a person skilled in the art. The solvent may be, for example, water, acetonitrile, an alcohol, and the like. Further, a concentration of the calibration substance (S1) in the solution A0 may be about 5 to 500 times a desired concentration of the calibration substance (S1) in the calibrant. Preferably, the concentration of the calibration substance (S1) in the solution A0 may be about 20 to 100 times the desired concentration of the calibration substance (S1) in the calibrant.

A solvent used for the solution B0 can be appropriately determined by a person skilled in the art. The solvent may be, for example, water, acetonitrile, an alcohol, and the like. The solvent may be used singly or in combination of two or more kinds of them. Any other substance may be added to the solvent. The solvent may include, for example, bovine serum albumin (BSA), ammonium bicarbonate, and the like. The solvent may contain, for example, an oxidation inhibitor or the like to maintain quality thereof. Proteins, amino acids, or reducing agents as described above may be used as the oxidation inhibitor.

Further, a concentration of the calibration substance (S2) in the solution B0 may be about 5 to 500 times a concentration of the calibration substance (S2) in the calibrant. Preferably, the concentration of the calibration substance (S2) in the solution B0 may be about 20 to 100 times the desired concentration of the calibration substance (S2) in the calibrant.

Next, a solvent (for example, water, acetonitrile, an alcohol, etc.) is added to the solution A0 to produce a solution A that contains the calibration substance (S1) at a desired concentration. Further, a solvent (for example, water, acetonitrile, an alcohol, etc.) and the solution B0 containing the calibration substance (S2) are added to the solution A0 to produce a solution B that contains the calibration substance (S1) at a concentration equal to the above desired concentration in the solution A, and further contains the calibration substance (S2). An amount of the solution B0 added to the solution A0 may be appropriately adjusted so that a peak intensity ratio of a ratio (S2/S1) of the calibration substance (S2) to the calibration substance (S1) is a predetermined value.

By producing the solution A and the solution B from the solution A0 as described above, the concentration of the calibration substance (S1) in the solution A and the concentration of the calibration substance (S1) in the solution B can be made more precisely the same. As a result, a concentration of the calibration substance (S1) in a calibrant to be produced in a preparation step that will be described later can be made more precisely constant.

Alternatively, the solution A0 may be diluted with a solvent in advance to produce a solution A0' that contains the calibration substance (S1) at a concentration closer to that of the calibration substance (S1) in the calibrant to be produced. The solution A and the solution B may be produced by using the solution A0' and the solution B0. By using the solution A0', an amount of the solvent added in preparation of the solution A and the solution B can be reduced. As a result, a concentration of the calibration substance (S1) in the calibrant to be produced in a preparation step that will be described later can be made further precisely constant.

### [3-2. Preparation step]

A calibrant in which a ratio (a concentration ratio S2/S 1) of, relative to a concentration of the calibration substance (S1), a concentration of the calibration substance (S2) is prepared to a predetermined value is produced by using the solution A and the solution B prepared in the previous step, wherein
the solution A comprises the calibration substance (S 1) at a predetermined concentration, and
the solution B comprises the calibration substance (S1) at a concentration equal to the above predetermined concentration, and further comprises the calibration substance (S2).

As the method of producing the calibrant using the solution A and the solution B, for example, the solution A and the solution B may be respectively measured so that a concentration ratio S2/S 1 in the calibrant is a predetermined concentration ratio, and mixed.

As described above, the concentrations of the calibration substance (S 1) in both of the solution A and the solution B are equal, and therefore the concentration of the calibration substance (S1) in the calibrant is constant irrespective of a mixing ratio (a content ratio) of the solution A and the solution B. In contrast, the concentration of the calibration substance (S2) in the calibrant can be prepared to any value depending on the mixing ratio (the content ratio) of the solution A and the solution B. That is, the concentration ratio S2/S 1 in the calibrant can be prepared to a predetermined value.

It is also possible to produce two or more calibrants which contains at least two calibration substances (S1) and (S2), and are respectively different in a ratio (a concentration ratio S2/S 1) of, relative to a concentration of the calibration substance (S1) in the calibrant, a concentration of the calibration substance (S2) in the calibrant. Hereinafter, explanation is made for the case of producing a plurality of calibrants which are constant in the concentration of the calibration substance (S1) in the calibrant and respectively different in the concentration of the calibration substance (S2) in the calibrant, thereby being respectively different in the concentration ratio S2/S 1 in the calibrant.

In this case, the concentration of the calibration substance (S2) in the solution B prepared in the previous step may be about 1.1 to 10 times the concentration of the calibration substance (S2) in the calibrant with the highest concentration of the calibration substance (S2) among the two or more calibrants. The concentration of the calibration substance (S2) in the solution B may be, for example, 1.2 to 5 times that in the calibrant with the highest concentration, and preferably, about 1.5 to 5 times that in the calibrant with the highest concentration. For example, the concentration of the calibration substance (S2) in the solution B may be twice that of the calibration substance (S2) in the calibrant with the highest concentration of the calibration substance (S2).

As described above, the concentrations of the calibration substance (S1) in both of the solution A and the solution B are equal, and therefore the concentration of the calibration substance (S1) in each of the calibrants is constant irrespective of a mixing ratio (a content ratio) of the solution A and the solution B. In contrast, the two or more calibrants which are different in the concentrations of the calibration substance (S2) in the calibrants can be made by varying the mixing ratio (the content ratio) of the solution A and the solution B. That is, concentration ratios S2/S1 in the two or more calibrants can be prepared to predetermined values which are respectively different.

For example, three calibrants (represented as IC-1, IC-2, and IC-3) may be produced. The solution A and the solution B may be mixed, respectively, so that a concentration ratio S2/S1 in each of IC-1, IC-2, and IC-3 is a different desired concentration ratio.

Alternatively, the solution A and the solution B may be mixed to obtain one calibrant, a mixing operation may be performed to separate a part of the one calibrant from the resulting one calibrant and further mix the separated part of the one calibrant with the solution A, and thereby another calibrant in which a ratio of, relative to a concentration of the one calibration substance (S1), a concentration of the another calibration substance (S2) is a different value from the concentration ratio in the one calibrant may be obtained. This mixing operation process may be sequentially repeated to obtain not less than three calibrants.

That is, the solution A and the solution B may be mixed to produce IC-1, and then a part of IC-1 and the solution A may be mixed to produce IC-2. Further, a part of the resulting IC-2 and the solution A may be mixed to produce IC-3. In this case, the concentration ratio S2/S1 is the highest in IC-1, and decreases in order of IC-2 and IC-3. That is, the solution B is sequentially diluted with the solution A.

Alternatively, the solution A and the solution B may be mixed to obtain one calibrant, a mixing operation may be performed to separate a part (for example, a half) of the one calibrant from the resulting one calibrant and further mix the separated part of the one calibrant with the solution A in an amount equal to the part (for example, the half) of the one calibrant separated, and thereby another calibrant in which a ratio of, relative to a concentration of the one calibration substance (S1), a concentration of the another calibration substance (S2) is 1/2 of the concentration ratio in the one calibrant may be obtained. This mixing operation process may be sequentially repeated (so called "two-fold serial dilutions") to obtain not less than three calibrants.

That is, the solution A and the solution B may be mixed to produce IC-1, and then a part (for example, a half) of IC-1 and the solution A in the same amount of the part of IC-1 may be mixed to produce IC-2. Further, a part (for example, a half) of the resulting IC-2 and the solution A in the same amount of the part of the resulting IC-2 may be mixed to produce IC-3. In this case, the concentration ratio S2/S1 in IC-1 is the highest, the concentration ratio S2/S1 in IC-2 is 1/2 of that in IC-1, and the concentration ratio S2/S1 in IC-3 is 1/2 of that in IC-2 (1/4 of that in IC-1). That is, the solution B is subjected to two-fold serial dilutions with the solution A.

Alternatively, the solution A and the solution B may be mixed to obtain one calibrant, a mixing operation may be performed to separate a part of the one calibrant from the resulting one calibrant and further mix the separated part of the one calibrant with the solution B, and thereby another calibrant in which a ratio of, relative to a concentration of the one calibration substance (S1), a concentration of the another calibration substance (S2) is a different value from the concentration ratio in the one calibrant may be obtained. This mixing operation process may be sequentially repeated to obtain not less than three calibrants.

That is, the solution A and the solution B may be mixed to produce IC-1, and then a part of IC-1 and the solution B may be mixed to produce IC-2. Further, a part of the resulting IC-2 and the solution B may be mixed to produce IC-3. In this case, the concentration ratio S2/S1 is the lowest in IC-1, and increases in order of IC-2 and IC-3.

Alternatively, the solution A and the solution B may be mixed to obtain one calibrant, a mixing operation may be performed to separate a part (for example, a half) of the one calibrant from the resulting one calibrant and further mix the separated part of the one calibrant with the solution B in an amount equal to the part (for example, the half) of the one calibrant separated, and thereby another calibrant in which a ratio of, relative to a concentration of the one calibration substance (S1), a concentration of the another calibration substance (S2) is different value from the concentration ratio in the one calibrant may be obtained.

That is, the solution A and the solution B may be mixed to produce IC-1, and then a part (for example, a half) of IC-1 and the solution B in the same amount of the part of IC-1 may be mixed to produce IC-2. Further, a part (for example, a half) of the resulting IC-2 and the solution B in the same amount of the part of the resulting IC-2 may be mixed to produce IC-3.

Fig. 1 is a conceptual diagram of a first embodiment of a preparation step, in the case of producing five calibrants (represented as IC-1, IC-2, IC-3, IC-4 and IC-5). Fig. 1 shows the case where the concentration ratios S2/S1 in IC-1, IC-2, IC-3, IC-4 and IC-5 are 4 (S2:S1 = 4:1), 2 (S2:S1 = 2:1), 1 (S2:S1 = 1:1), 1/2 (S2:S1 = 0.5:1), and 1/4 (S2:S1 = 0.25:1), respectively.

As shown in Fig. 1, the first embodiment is a method in which the solution B containing the calibration substance (S1) and the calibration substance (S2) is serially diluted two-fold with the solution A containing the calibration substance (S1) to produce IC-1, IC-2, IC-3, IC-4 and IC-5.

More specifically, the solution B is mixed with the solution A in a predetermined ratio (for example, in equal amounts) to produce IC-1. Next, a part (for example, a half) of the produced IC-1 is mixed with the same amount of the solution A to produce IC-2. A part (for example, a half) of the produced IC-2 is mixed with the same amount of the solution A to produce IC-3. A part (for example, a half) of the produced IC-3 is mixed with the same amount of the solution A to produce IC-4. Finally, a part (for example, a half) of the produced IC-4 is mixed with the same amount of the solution A to produce IC-5. A plurality of calibrants which are respectively different in a ratio (a concentration ratio S2/S1) of a concentration of the calibration substance (S2) relative to a concentration of the calibration substance (S1) may be produced, by using such a method of conducting sequential dilutions as described above.

Fig. 2 is a conceptual diagram of a second embodiment of a preparation step, in the case of producing five calibrants (represented as IC-1, IC-2, IC-3, IC-4 and IC-5). As in the case of Fig. 1, Fig. 2 also shows the case where the concentration ratios S2/S1 in IC-1, IC-2, IC-3, IC-4 and IC-5 are 4 (S2:S1 = 4:1), 2 (S2:S1 = 2:1), 1 (S2:S1 = 1:1), 1/2 (S2:S1 = 0.5:1), and 1/4 (S2:S1 = 0.25:1), respectively.

In the second embodiment, a solution D containing the calibration substance (S1) and a solution E containing the calibration substance (S2) are prepared.

As shown in Fig. 2, the second embodiment is a method in which the solution E containing the calibration substance (S2) is serially diluted two-fold with a solvent of the solution E to produce five solutions, and then the solution D containing the calibration substance (S1) is added to each of the five solutions to produce IC-1, IC-2, IC-3, IC-4 and IC-5.

More specifically, the solution E is mixed with the solvent in a predetermined ratio (for example, in equal amounts) to produce a solution E1. Next, a part (for example, a half) of the produced solution E1 is mixed with the same amount of the solvent to produce a solution E2. A part (for example, a half) of the produced solution E2 is mixed with the same amount of the solvent to produce a solution E3. A part (for example, a half) of the produced solution E3 is mixed with the same amount of the solvent to produce a solution E4. A part (for example, a half) of the produced solution E4 is mixed with the same amount of the solvent to produce a solution E5. Then, the solution D is added to each of the solution E1, the solution E2, the solution E3, the solution E4, and the solution E5 to produce IC-1, IC-2, IC-3, IC-4 and IC-5, respectively. A plurality of calibrants which are respectively different in the concentration ratio S2/S1 may be produced, by using such a method as described above.

In the case of using the solution D containing the calibration substance (S1) and the solution E containing the calibration substance (S2) as in the second embodiment, the solution D containing the calibration substance (S1) is measured and added to each of the solutions (E1, E2, E3, E4, and E5) which are respectively different in the concentration of the calibration substance (S2). In such a production method, in addition to an error in the concentration of the calibration substance (S2) due to a measuring error when diluting the solution E with the solvent, an error in the concentration of the calibration substance (S 1) exists due to a measuring error when mixing E1, E2, E3, E4, and E5, respectively, with the solution D. As a result, it is concerned that the concentration ratio S2/S1 of each of the calibrants may deviate from the desired value.

On the other hand, it is possible to minimize a variation in the concentration of the calibration substance (S1) among the two or more calibrants by using the first embodiment. As a result, it is possible to produce the two or more calibrants so that the concentration ratio S2/S1 of each of the calibrants is precisely the desired value. By using the calibrants produced in this way, accuracy of a calibration formula can be higher when a calibration formula in a mass spectrometer is calculated by the method that will be described later.

The preparation step using the solution A and the solution B may be performed by volume measuring. The preparation step using the solution A and the solution B may be performed by weight measuring.

In the preparation step, required amounts of the solution A and the solution B are respectively measured and mixed. Alternatively, required amounts of a calibrant prepared by mixing and the solution A are respectively measured and mixed. Measuring at this time may be done by volume by pipetting or other means. Measuring at this time may be done by weight using an electronic balance or the like.

Particularly, when at least one of the solution A and the solution B has viscosity, use of an electronic balance reduces variation in measuring caused by a pipet or a manual technique of an operator, and therefore the concentration of the calibration substance (S2) in the calibrant can be made to the desired value with higher accuracy. As a result, the concentration ratio S2/S1 can be prepared to the desired value with higher accuracy.

In the case of using the electronic balance, accurate work records can be kept, which is preferable from the viewpoint of traceability. Further, it is easy to scale up the amount of calibrant production.

In addition to the two calibration substances (S1) and (S2), one or more other substances may be included in the calibrant as calibration substances. For example, when a calibrant further contains a calibration substance S3, a calibrant may be produced so that a ratio (a concentration ratio S2/S 1) of the calibration substance (S2) to the calibration substance (S1) and a ratio (a concentration ratio S3/S1) of the calibration substance (S3) to the calibration substance (S1) may be prepared to respective predetermined values.

The calibrant produced in the previous step and the preparation step described above may be used as it is for measurement in a mass spectrometer. Alternatively, the calibrant may be diluted by a predetermined magnification when being used for measurement in the mass spectrometer. A dilution magnification may be, for example, about 2 to 50 times. The dilution magnification may be, for example, 10 times. A diluting solvent may appropriately be determined by those skilled in the art. The solvent contained in the calibrant may be used as the diluting solvent.

### [4. Kit]

An embodiment of a kit according to the present invention includes a plurality of calibrants which comprises not less than two calibration substances, wherein
the plurality of calibrants are respectively different in a concentration of at least one calibration substance of the calibration substances.

For example, when the calibration substances are represented as S1 and S2, a kit may include a plurality of calibrants which are different in a ratio S2/S1 of, relative to a concentration of one calibration substance (S1), a concentration of the other calibration substance (S2). The concentration ratios S2/S1 may be, for example, in a range of 1/4 to 4. For example, a kit may include five kinds of calibrant solutions in which the concentration ratios S2/S1 are adjusted to 1/4, 1/2, 1, 2, and 4, respectively. For example, in the five kinds of the calibrant solutions, a stable isotope-labeled Aβ1-38 (SIL-Aβ1-38) may be used as the calibration substance (S1), and Aβ1-38 may be used as the calibration substance (S2).

The calibrant included in the kit may be used as it is for measurement in a mass spectrometer. Alternatively, the calibrant included in the kit may be diluted by a predetermined magnification and the diluted calibrant may be used for measurement in the mass spectrometer.

In each of the plurality of the calibrant included in such a kit, the concentration ratio S2/S1 may be precisely adjusted. By using the kit, an operator can measure, in the mass spectrometer, the plurality of the calibrants in which the concentration ratios S2/S1 are precisely adjusted, respectively. As a result, calculation of a calibration formula in the mass spectrometer as described later can be conducted with high accuracy.

Another embodiment of a kit according to the present invention includes not less than two calibration substances.

The kit includes materials used for producing a calibrant that is prepared so that a concentration ratio S2/S1 of, relative to one calibration substance (S1), another calibration substance (S2) is a desired value.

By using such a kit, an operator can produce a calibrant that is prepared so that a concentration ratio S2/S1 is a desired value when calculating a calibration formula in a mass spectrometer. By using such a kit, the operator can prevent change with time of the concentration ratio S2/S1 caused by storing the calibrant. As a result, calculation of a calibration formula in the mass spectrometer as described later can be conducted with high accuracy.

The kit may further include a solvent used for the calibrant.

The kit may further include a container such as an ampule to accommodate the calibrant.

### [5. Calibration of machine difference of mass spectrometers]

A method for calibrating a difference between mass spectrometers by using the calibrant according to this embodiment will be described in detail below. A calibrant is measured by a mass spectrometer, and a calibration formula for said mass spectrometer is calculated using the measurement results. A sample containing an analyte substance is measured in said mass spectrometer, and then the measurement results of the sample is calibrated by using the calculated calibration formula. This calibration makes it possible to perform a comparative evaluation of the measurement results of the analyte substances between two or more samples irrespective of a difference between mass spectrometer machines.

### [5-1. Analyte substances]

The analyte substances to be analyzed are not particularly limited, and examples thereof include peptides, glycopeptides, glycans, proteins, lipids, glycolipids, and low molecular medicines. The peptides, glycopeptides, sugar chains, proteins, lipids, glycolipids, and low molecular medicines include those of various kinds. More specifically, the analyte substances may be Aβ and an Aβ related peptide. The "Aβ and an Aβ related peptide" may simply collectively be called "Aβ related peptides". The "Aβ and an Aβ related peptide" includes Aβ generated by cleaving amyloid precursor protein (APP) and peptides containing even part of the sequence of Aβ.

For example, Aβ related peptides includes the followings.

APP677-709 (Aβ6-38) (SEQ ID NO.: 1):
   HDSGYEVHHQKLVFFAEDVGSNKGAIIGLMVGG
APP672-704 (Aβ1-33) (SEQ ID NO.: 2):
   DAEFRHDSGYEVHHQKLVFFAEDVGSNKGAIIG
APP677-711 (Aβ6-40) (SEQ ID NO.: 3):
   HDSGYEVHHQKLVFFAEDVGSNKGAIIGLMVGGW
APP672-706 (Aβ1-35) (SEQ ID NO.: 4):
   DAEFRHDSGYEVHHQKLVFFAEDVGSNKGAIIGLM
APP672-708 (Aβ1-37) (SEQ ID NO.: 5):
   DAEFRHDSGYEVHHQKLVFFAEDVGSNKGAIIGLMVG
APP674-711 (Aβ3-40) (SEQ ID NO.: 6):
   EFRHDSGYEVHHQKLVFFAEDVGSNKGAIIGLMVGGW
APP672-711 (Aβ1-40) (SEQ ID NO.: 7):
   DAEFRHDSGYEVHHQKLVFFAEDVGSNKGAIIGLMVGGW
OxAPP672-711 (OxAβ1-40) (SEQ ID NO.: 8):
   DAEFRHDSGYEVHHQKLVFFAEDVGSNKGAIIGLMVGGVV (Met 706 is oxidized)
APP672-713 (Aβ1-42) (SEQ ID NO.: 9):
   DAEFRHDSGYEVHHQKLVFFAEDVGSNKGAIIGLMVGGWIA
APP669-711 (SEQ ID NO.: 10):
   VKMDAEFRHDSGYEVHHQKLVFFAEDVGSNKGAIIGLMVGGVV
APP672-709 (Aβ1-38) (SEQ ID NO.: 11):
   DAEFRHDSGYEVHHQKLVFFAEDVGSNKGAIIGLMVGG
APP672-710 (Aβ1-39) (SEQ ID NO.: 12):
   DAEFRHDSGYEVHHQKLVFFAEDVGSNKGAIIGLMVGGV

Amyloid precursor protein (APP) is a single-pass transmembrane protein and is composed of 770 amino acid residues. Amyloid precursor protein (APP) is proteolyzed by β secretase and y secretase, resulting in the production of amyloid β peptide (Aβ). APP672-713 and Aβ1-42 indicate the same peptide (SEQ ID NO.: 9). APP672-711 and A1β-40 indicate the same peptide (SEQ ID NO.: 7).

Among the above-described Aβ related peptides, Aβ1-42 (SEQ ID NO.: 9), Aβ1-40 (SEQ ID NO.: 7), APP669-711 (SEQ ID NO.: 10), and Aβ1-39 (SEQ ID NO.: 12) are effective biomarkers regarding Alzheimer's disease.

A ratio of APP669-711 level to APP672-713 (Aβ1-42) level: APP669-711/APP672-713 (Aβ1-42);
a ratio of APP672-711 (Aβ1-40) level to APP672-713 (Aβ1-42) level: APP672-711 (Aβ1-40)/APP672-713 (Aβ1-42); and
a ratio of APP672-710 (Aβ1-39) level to APP672-713 (Aβ1-42) level: APP672-710 (Aβ1-39)/APP672-713 (Aβ1-42) are also examples of effective biomarkers regarding Alzheimer's disease.

Also, the peptides may be those obtained by immunoprecipitation (IP). Alternatively, the peptides may be those generated by digestion of protein with an enzyme such as peptidase, or those fractionated by chromatography.

The analyte substances may include an internal standard substance. The internal standard substance may appropriately be selected by those skilled in the art. For example, a substance labeled with a stable isotope may be used. One substance of the analyte substances may be labeled with a stable isotope. In Examples, examples using a stable isotope-labeled Aβ1-38 (SIL-Aβ1-38) as an internal standard substance are shown.

A sample containing the analyte substances is subjected to mass spectrometry. The sample to be subjected to mass spectrometry is not particularly limited, and may be, for example, a living body-derived sample. The living body-derived sample includes body fluids such as blood, cerebrospinal fluid (CSF), urine, body secreting fluid, saliva, and sputum; and feces. The blood sample includes whole blood, plasma, serum and the like. The blood sample can be prepared by appropriately treating whole blood collected from an individual. The treatment performed in the case of preparing a blood sample from collected whole blood is not particularly limited, and any treatment that is clinically acceptable may be performed. For example, centrifugal separation or the like may be performed. The blood sample to be subjected to mass spectrometry may be appropriately stored at a low temperature such as freezing in the intermediate stage of the preparation step or in the post stage of the preparation step. In the present invention, when the living body-derived sample such as a blood sample is subjected to mass spectrometry, the living body-derived sample is disposed of rather than being returned to the individual subject from which it is derived.

The sample to be subjected to mass spectrometry may be one that has been subjected to various pretreatments. For example, the sample may be one that has been subjected to immunoprecipitation (IP). The sample may be one that has been subjected to protein digestion with an enzyme such as peptidase. The sample may be one that has been subjected to chromatography. The sample to be subjected to mass spectrometry may be one to which a certain amount of an internal standard substance has been added.

As the sample to be subjected to mass spectrometry, an eluate obtained by immunoprecipitation previously performed may be subjected to mass spectrometry (Immunoprecipitation-mass spectrometry; IP-MS). The immunoprecipitation may be performed using an antibody-immobilized carrier prepared using immunoglobulin having an antigen-binding site that can recognize an analyte substance or an immunoglobulin fragment containing an antigen-binding site that can recognize an analyte substance.

Consecutive immunoprecipitation (cIP) may be conducted on the sample to be subjected to mass spectrometry, and then a peptide in the sample may be detected by a mass spectrometer (cIP-MS). By conducting affinity purification twice consecutively, impurities that cannot be excluded by one affinity purification can be further reduced by the second affinity purification. Therefore, it is possible to prevent the ionization suppression of polypeptide due to impurities, and it becomes possible to measure even a very small amount of polypeptide in a living body sample with high sensitivity by mass spectrometry.

### [5-2. Mass spectrometry]

The mass spectrometry is not particularly limited, and examples thereof include those for mass spectrometry such as matrix-assisted laser desorption/ionization (MALDI) mass spectrometry or electrospray ionization (ESI) mass spectrometry. For example, a MALDI-TOF (matrix-assisted laser desorption/ionization time-of-flight) mass spectrometer, a MALDI-IT (matrix-assisted laser desorption/ionization ion trap) mass spectrometer, a MALDI-IT-TOF (matrix-assisted laser desorption/ionization ion trap time-of-flight) mass spectrometer, a MALDI-FTICR (matrix-assisted laser desorption/ionization Fourier transform ion cyclotron resonance) mass spectrometer, an ESI-QqQ (electrospray ionization triple quadrupole) mass spectrometer, an ESI-Qq-TOF (electrospray ionization tandem quadrupole time-of-flight) mass spectrometer, or an ESI-FTICR (electrospray ionization Fourier transform ion cyclotron resonance) mass spectrometer or the like can be used.

A matrix and a matrix solvent can be appropriately determined by a person skilled in the art depending on the analyte substance.

As the matrix, for example, α-cyano-4-hydroxycinnamic acid (CHCA), 2,5-dihydroxybenzoic acid (2,5-DHB), sinapinic acid, 3-aminoquinoline (3-AQ) or the like can be used.

The matrix solvent can be selected from the group consisting of, for example, acetonitrile (ACN), trifluoroacetic acid (TFA), methanol, ethanol and water, and used. More specifically, an ACN-TFA aqueous solution, an ACN aqueous solution, methanol-TFA aqueous solution, a methanol aqueous solution, an ethanol-TFA aqueous solution, an ethanol solution or the like can be used. The concentration of ACN in the ACN-TFA aqueous solution can be, for example, 10 to 90% by volume, the concentration of TFA can be, for example, 0.05 to 1% by volume, preferably 0.05 to 0.1% by volume.

The matrix concentration can be, for example, 0.1 to 50 mg/mL, preferably 0.1 to 20 mg/mL, or 0.3 to 20 mg/mL, further preferably 0.5 to 10 mg/mL.

In the case of employing MALDI mass spectrometry as a detecting system, a matrix additive (comatrix) is preferably used together. The matrix additive can be appropriately selected by a person skilled in the art depending on the analysis subject (polypeptides) and/or the matrix. For example, as the matrix additive, a phosphonic acid group-containing compound can be used. Specific examples of a compound containing one phosphonic acid group include phosphonic acid, methylphosphonic acid, phenylphosphonic acid, 1-naphthylmethylphosphonic acid, and the like. Specific examples of a compound containing two or more phosphonic acid groups include methylenediphosphonic acid (MDPNA), ethylenediphosphonic acid, ethane-1-hydroxy-1,1-diphosphonic acid, nitrilotriphosphonic acid, ethylenediaminetetraphosphonic acid, and the like. Among the aforementioned phosphonic acid group-containing compounds, compounds having two or more, preferably two to four phosphonic acid groups in one molecule are preferred.

The use of the phosphonic acid group-containing compound is useful, for example, when metal ions of the washing solution remaining on the surface of the antibody-immobilizing carrier are contaminated into the eluate after the dissociating step. The metal ions adversely affect on the background in the mass spectrometry. The use of the phosphonic acid group-containing compound is effective for suppressing such an adverse effect.

Besides the aforementioned matrix additive, a more common additive, for example, a substance that is selected from the group consisting of ammonium salts and organic bases may be used.

The matrix additive can be prepared as a solution of 0.1 to 10 w/v%, preferably 0.2 to 4 w/v% in water or in a matrix solvent. The matrix additive solution and the matrix solution can be mixed in a volume ratio of, for example, 1 : 100 to 100 : 1, preferably 1 : 10 to 10 : 1.

### [5-3. Calibration of measurement results in mass spectrometer]

A calibration formula in a mass spectrometer can be calculated using the calibrant according to this embodiment. The calibrant according to this embodiment includes not less than two calibration substances, wherein a ratio of, relative to a concentration of one calibration substance of the not less than two calibration substances, a concentration of another calibration substance of the not less than two calibration substances is a predetermined value. The measurement results of the calibrant according to this embodiment by a mass spectrometer are used to calculate a calibration formula for the mass spectrometer. Then, the measurement results of analyte substances are calibrated using the calculated calibration formula. For example, the calibrant according to this embodiment may be used for a calibration method using a calibration formula calculated using a standard machine. Further, the measurement results of mass spectrometry can be standardized using a peak intensity ratio of two calibration substances whose concentration ratio is a predetermined value.

The calibration formula is calculated for every mass spectrometer. Even in the case of the same mass spectrometer, a new calibration formula is preferably calculated when a part such as a detector or the like is exchanged or when the setting of the machine, such as a detector voltage or the like, is changed. Alternatively, the calibration formula may regularly be calculated. This makes it possible to detect the deterioration or failure of a detector or the like of the mass spectrometer or to obtain measurement results from which the influence thereof has been removed. Therefore, it is possible to perform a comparative evaluation of abundance ratios of analyte substances between two or more samples with high accuracy irrespective of a difference between mass spectrometer machines or the machine conditions of the mass spectrometer.

More preferably, when analyte substances are measured, a calibration formula is calculated by measuring calibration substances under the same machine conditions as the measurement of the analyte substances. This makes it possible to use a calibration formula determined under the same conditions as the measurement of the analyte substances and therefore to further improve the accuracy of calibration. Therefore, it is possible to perform a comparative evaluation of abundance ratios of analyte substances between two or more samples with higher accuracy irrespective of a difference between mass spectrometer machines or the machine conditions of a mass spectrometer.

### [5-3-1. Calibration using calibration formula using standard machine]

### [5-3-1-1. Calculation of calibration formula]

A calibrant solution containing not less than two calibration substances is measured by a standard machine to obtain a signal peak intensity of each of the calibration substances. In the calibrant solution, a ratio of, relative to a concentration of one calibration substance of the not less than two calibration substances, a concentration of another calibration substance of the not less than two calibration substances is a predetermined value. The ratio of, relative to the signal peak intensity of one of the calibration substances, the signal peak intensity of each of the one or more other calibration substances is calculated.

A plurality of calibrants which are respectively different in a ratio of, relative to a concentration of one calibration substance of the not less than two calibration substances, a concentration of another calibration substance of the not less than two calibration substances may be used to obtain two or more signal peak intensity ratios. Alternatively, one calibrant containing not less than three calibration substances may be used to obtain two or more signal peak intensity ratios.

As the standard machine, a mass spectrometer having high reliability is preferably used. Each user can freely set a mass spectrometer that should be used as a standard machine.

The same calibrant solution as the calibrant solution measured by the standard machine is measured by a mass spectrometer, for which a calibration formula should be calculated, to obtain a signal peak intensity of each of the calibration substances. The ratio of, relative to the signal peak intensity of one of the calibration substances, the signal peak intensity of each of the one or more other calibration substances is calculated.

A regression equation is calculated between the signal peak intensity ratio calculated by the standard machine and the signal peak intensity ratio calculated by the mass spectrometer for which a calibration formula should be calculated. The calculation of a regression equation may appropriately be performed using a known method such as a least-square method or the like. The calculation of a regression equation may be performed using the logarithms of the signal peak intensity ratios. The regression equation may appropriately be selected from among a linear regression equation, a multiple regression equation, an exponential regression equation, a logarithmic regression equation, and a power regression equation.

For example, a linear regression equation may be calculated using values obtained by logarithmic transformation of the signal peak intensity ratios. Alternatively, for example, a power regression equation may be calculated using the signal peak intensity ratios. The calculated regression equation is defined as a calibration formula for the said mass spectrometer.

For example, when the logarithm of the signal peak intensity ratio calculated by the mass spectrometer for which a calibration formula should be calculated is defined as x, and the logarithm of the signal peak intensity ratio calculated by the standard machine is defined as y, calibration coefficients a and b of:
a linear regression equation (y = ax + b)
can be calculated. The calculated linear regression equation (y = ax + b) can be used as a calibration formula for the said mass spectrometer.

Alternatively, when the signal peak intensity ratio calculated by the mass spectrometer for which a calibration formula should be calculated is defined as x, and the signal peak intensity ratio calculated by the standard machine is defined as y, calibration coefficients a and b of:
a power regression equation (y = ax^{b})
may be calculated. The calculated power regression equation (y = ax^{b}) can be used as a calibration formula for the said mass spectrometer.

The calibration coefficients a and b are considered as values inherent in the said mass spectrometer. Therefore, the calibration coefficients a and b may vary with a difference between mass spectrometer machines or the machine conditions of the mass spectrometer. The calibration coefficient b is more strongly influenced by a difference between mass spectrometer machines or the machine conditions of the mass spectrometer than the calibration coefficient a. Therefore, in, for example, a power regression equation (y = ax^{b}), the value a may be fixed to 1 (a = 1) so that only the value b is calculated and adopted as a calibration coefficient. That is, y = x^{b} may be used as a calibration formula instead of the power regression equation (y = ax^{b}). In such a case where only b is used as a calibration coefficient, the value b is referred to as a calibration value.

### [5-3-1-2. Measurement of analyte substances and calibration]

A sample containing analyte substances is measured by the mass spectrometer for which a calibration formula has been calculated in 5-3-1-1. to obtain a signal peak intensity of each of the analyte substances. One of the analyte substances (e.g., an internal standard substance) is used as a reference to calculate the ratio of, relative to the signal peak intensity of the analyte substance as a reference, the signal peak intensity of another analyte substance.

The calculated signal peak intensity ratio is calibrated using the calibration formula calculated above in 5-3-1-1. The signal peak intensity ratio after calibration is a value in which a machine difference from the standard machine is cancelled. That is, the signal peak intensity ratio after calibration is equivalent to a signal peak intensity ratio obtained by measurement using the standard machine. Therefore, the use of signal peak intensity ratios after calibration makes it possible to perform a comparative evaluation of the signal peak intensity ratios of the analyte substances, that is, the abundance ratios of the analyte substances between two or more samples irrespective of a difference between mass spectrometer machines.

### [5-3-2. Calibration by standardizing signal peak intensity ratio (intensity ratio calibration)]

### [5-3-2-1. Calculation of calibration formula]

A calibration formula for standardizing a signal peak intensity ratio can be calculated using the calibrant according to this embodiment, that is, a plurality of calibrant solutions which are respectively different in concentration ratios of two calibration substances.

Two or more calibrant solutions (intensity ratio calibrants; ICs) whose concentration ratio of two calibration substances is known are used. Two or more solutions may be used which contain one calibration substance at a certain concentration and contain the other calibration substance at different concentrations. For example, solutions may be used which contain SIL-Aβ1-38 at a certain concentration and contain Aβ1-38 at different concentrations so that the concentration ratios of Aβ1-38 to SIL-Aβ1-38 are adjusted to 1/4, 1/2, 1, 2, and 4.

Two or more calibrant solutions (intensity ratio calibrants; ICs) whose concentration ratio of two calibration substances is known are measured by a mass spectrometer for which a calibration formula should be calculated to obtain a signal peak intensity of each of the calibration substances in each of the solutions. For each of the solutions, the ratio of, relative to the signal peak intensity of the calibration substance contained at a certain concentration, the signal peak intensity of the other calibration substance is calculated.

A regression equation is calculated between the known concentration ratio of the two calibration substances in each of the calibrant solutions and the signal peak intensity ratio calculated above. The calculation of a regression equation may appropriately be performed using a known method such as a least-square method or the like. The calculation of a regression equation may be performed using the logarithms of the signal peak intensity ratios. The regression equation may appropriately be selected from among a linear regression equation, a multiple regression equation, an exponential regression equation, a logarithmic regression equation, and a power regression equation.

For example, a linear regression equation may be calculated using the logarithms of the signal peak intensity ratios. Alternatively, a power regression equation may be calculated using the signal peak intensity ratios. The calculated regression equation is defined as a calibration formula for the said mass spectrometer.

For example, when the logarithm of the known concentration ratio of the two calibration substances in each of the solutions is defined as x, and the logarithm of the signal peak intensity ratio calculated by the mass spectrometer for which a calibration formula should be calculated is defined as y, calibration coefficients a and b of:
a linear regression equation (y = ax + b)
can be calculated. The calculated linear regression equation (y = ax + b) can be used as a calibration formula for the said mass spectrometer.

Alternatively, when the known concentration ratio of the two calibration substances in each of the solutions is defined as x, and the signal peak intensity ratio calculated by the mass spectrometer for which a calibration formula should be calculated is defined as y, calibration coefficients a and b of:
a power regression equation (y = ax^{b})
may be calculated. The calculated power regression equation (y = ax^{b}) can be used as a calibration formula for the said mass spectrometer.

The calibration coefficients a and b are considered as values inherent in the said mass spectrometer. Therefore, the calibration coefficients a and b may vary with a difference between mass spectrometer machines or the machine conditions of the mass spectrometer. The calibration coefficient b is more strongly influenced by a difference between mass spectrometer machines or the machine conditions of the mass spectrometer than the calibration coefficient a. Therefore, in, for example, a power regression equation (y = ax^{b}), the value a may be fixed to 1 (a = 1) so that only the value b is calculated and adopted as a calibration coefficient. That is, y = x^{b} may be used as a calibration formula instead of the power regression equation (y = ax^{b}). In such a case where only b is used as a calibration coefficient, the value b is referred to as a calibration value.

### [5-3-2-2. Measurement of analyte substances and calibration]

A sample containing analyte substances is measured by the mass spectrometer for which a calibration formula has been calculated in 5-3-2-1. to obtain a signal peak intensity of each of the analyte substances. One of the analyte substances (e.g., an internal standard substance) is used as a reference to calculate the ratio of, relative to the signal peak intensity of the analyte substance as a reference, the signal peak intensity of another analyte substance.

The calculated signal peak intensity ratio is calibrated using the calibration formula calculated above in 5-3-2-1. The signal peak intensity ratio of the analyte substances is converted by calibration to a signal peak intensity ratio standardized by the calibration substances. In the obtained standardized signal peak intensity, a machine difference is cancelled by the calibration formula. Therefore, it is possible to perform a comparative evaluation of abundance ratios of the analyte substances between two or more samples irrespective of a difference between mass spectrometer machines. Specifically, it is possible to perform direct comparison with the measurement results of mass spectrometry obtained in not only Japan but also other countries such as America and France. Further, this calibration technique is a versatile technique applicable to various researches and examinations using mass spectrometry.

### [5-3-2-3. Calibration value and machine conditions]

As described above, calibration is performed by calculating a calibration formula for a mass spectrometer used for measurement of analyte substances under the same machine conditions as the measurement of the analyte substances, which makes it possible to, as described above, perform a comparative analysis of the abundance ratios of the analyte substances between two or more samples irrespective of a difference between mass spectrometer machines or the machine conditions of the mass spectrometer.

As described above, the calibration formula for intensity ratio calibration can be prepared using two or more calibrant solutions whose concentration ratio of two calibration substances is known. For example, five kinds of solutions may be used which contain SIL-Aβ1-38 at a certain concentration and contain Aβ1-38 at different concentrations so that the concentration ratios of Aβ1-38 to SIL-Aβ1-38 are adjusted to 1/4, 1/2, 1, 2, and 4. In the mass spectrum of each of the solutions, the peak of a certain amount of SIL-Aβ1-38 and the peak of Aβ1-38 that depends on the concentration thereof appear. Ideally, the peak intensity ratios are expected to correspond to the concentration ratios in the respective calibrant solutions. However, the peak intensity ratios vary depending on machine conditions, and therefore a calibration formula is calculated so that the peak intensity ratios correspond to the concentration ratios in the respective calibrant solutions, and the measurement results of analytes are calibrated. This makes it possible to cancel a machine difference.

The calibration formula varies with a difference between mass spectrometer machines. Further, even in the case of the same mass spectrometer, when a part such as a detector or the like is exchanged or a machine setting, such as a detector voltage or the like, is changed, conditions of the mass spectrometer are changed, and therefore the calibration formula varies depending on machine conditions. Further, also when machine conditions are changed due to, for example, deterioration of a detector or the like, the calibration formula may be changed.

For example, when the same sample is measured by a mass spectrometer, a signal peak intensity ratio detected by the mass spectrometer increases due to the influence of machine conditions such as deterioration of a detector or the like. The reason for this is considered to be that the signal peak of a calibrant substance whose abundance is low becomes small due to the influence of machine conditions such as deterioration of a detector or the like. Generally, when a signal peak intensity is excessively low in a mass spectrometer, measurement accuracy reduces from the viewpoint of S/N ratio. Therefore, it is preferred that the signal peak intensity is not excessively low in the mass spectrometer.

For example, in a case where a power regression equation (y = ax^{b}) is determined in 5-3-2-1., when a signal peak intensity detected by a mass spectrometer becomes low due to the influence of some kind of machine conditions, the calibration value b in the calibration formula increases. Therefore, by allowing the value b to fall within a certain range, the signal peak intensity is prevented from becoming excessively low, and therefore the measurement accuracy of the mass spectrometer further improves so that the accuracy of calibration further improves.

The value b can be varied by changing the detection sensitivity of the mass spectrometer. The value b can be controlled by, for example, changing a detector voltage. Alternatively, the value b may be changed by, for example, changing the baseline level of an analog digital (AD) converter.

### EXAMPLES

Hereinbelow, the present invention will be described more specifically with reference to examples, but is not limited to these examples.

### [Example 1: Production of calibrant on volume basis]

Fig. 3 is a schematic diagram showing a production method of a calibrant according to Example 1. As Example 1, five kinds of calibrants IC-1, IC-2, IC-3, IC-4, and IC-5 were produced which contain Aβ1-38 (SEQ ID NO.: 11) and a stable isotope-labeled Aβ1-38 (SIL-Aβ1-38) as calibration substances and are respectively different in a ratio (a concentration ratio Aβ1-38/SIL-Aβ1-38) of a concentration of Aβ1-38 to a concentration of SIL-Aβ1-38. The calibrants in Example 1 were produced as follows.

In Example 1, a concentration of SIL-Aβ1-38 in each of five kinds of calibrants IC-1, IC-2, IC-3, IC-4, and IC-5 is constant, and a concentration of Aβ1-38 in each of five kinds of calibrants IC-1, IC-2, IC-3, IC-4, and IC-5 is different.

### [1-1 Preparation of solution A and solution B]

As SIL-Aβ1-38, a product of AnaSpec (San Jose, CA, USA) was used. In SIL-Aβ1-38, carbon atoms in Phe and Ile are substituted by ¹³C. SIL-Aβ1-38 was dissolved in a solvent (20% acetonitrile aqueous solution containing 1 mg/mL BSA and 50 mM ammonium bicarbonate) to prepare a solution A0 (6-peptide-A solution). The concentration of SIL-Aβ1-38 in the solution A0 (6-peptide-A solution) was 100 nmol/L. The solution A0 further contained Aβ1-33 (300 nmol/L), Aβ1-34 (200 nmol/L), Aβ1-36 (100 nmol/L), Aβ1-37 (50 nmol/L), and APP669-711 (50 nmol/L).

Aβ1-38 was dissolved in the solvent (20% acetonitrile aqueous solution containing 1 mg/mL BSA and 50 mM ammonium bicarbonate) to prepare a solution B0 (Aβ1-38 solution). The concentration of Aβ1-38 in the solution B0 (Aβ1-38 solution) was 800 nM (nmol/L).

30 µL of the solution A0 (6-peptide-A solution) was added to 1170 µL of a solvent for IC concentrated solutions (20% acetonitrile aqueous solution containing 0.1 mg/mL BSA and 50 mM ammonium bicarbonate) to prepare a solution A (6-peptide-B). The concentration of SIL-Aβ1-38 in the solution A (6-peptide-B) was 2.5 nmol/L. The concentration of SIL-Aβ1-38 in the solution A (6-peptide-B) was adjusted to a desired SIL-Aβ1-38 concentration in the five calibrants IC-1, IC-2, IC-3, IC-4, and IC-5 to be produced.

The solvent for IC concentrated solutions is a liquid that are volatile because of containing acetonitrile, and are also viscous because of containing BSA.

10 µL of the solution A0 (6-peptide-A solution) and 10 µL of the solution B0 (Aβ1-38 solution) were added to 380 µL of the solvent for IC concentrated solutions (20% acetonitrile aqueous solution containing 0.1 mg/mL BSA and 50 mM ammonium bicarbonate) to prepare a solution B (IC-0). The concentration of SIL-Aβ1-38 in the solution B (IC-0) was 2.5 nmol/L that is equal to the concentration of SIL-Aβ1-38 in the solution A (6-peptide-B). The concentration of Aβ1-38 in the solution B (IC-0) was 20 nmol/L.

All measuring in 1-1 of Example 1 was done by volume by pipetting. A pipette of Eppendorf Research Plus was used.

### [1-2 Preparation of calibrants]

Next, the prepared solution B was serially diluted two-fold with the solution A to produce IC-1, IC-2, IC-3, IC-4, and IC-5 in the following manner.

200 µL of the prepared solution A and 200 µL of the prepared solution B were mixed to produce IC-1 solution. The ratio (the concentration ratio Aβ1-38/SIL-Aβ1-38) of the concentration of Aβ1-38 to the concentration of SIL-Aβ1-38 in the IC-1 solution was 4. 200 µL of the produced IC-1 solution and 200 µL of the solution A were mixed to produce IC-2 solution. The concentration ratio Aβ1-38/SIL-Aβ1-38 in the IC-2 solution was 2. 200 µL of the produced IC-2 solution and 200 µL of the solution A were mixed to produce IC-3 solution. The concentration ratio Aβ1-38/SIL-Aβ1-38 in the IC-3 solution was 1. 200 µL of the produced IC-3 solution and 200 µL of the solution A were mixed to produce IC-4 solution. The concentration ratio Aβ1-38/SIL-Aβ1-38 in the IC-4 solution was 1/2. 200 µL of the produced IC-4 solution and 200 µL of the solution A were mixed to produce IC-5 solution. The concentration ratio Aβ1-38/SIL-Aβ1-38 in the IC-5 solution was 1/4.

All measuring in 1-2 of Example 1 was done by volume by pipetting. A pipette of Eppendorf Research Plus was used. Accordingly, all measuring in Example 1 was done by volume by pipetting.

Each of three operators produced calibrants IC-1, IC-2, IC-3, IC-4, and IC-5 three times following the above procedure. The produced calibrants were preserved by freezing. Each of the produced calibrants was diluted ten-fold and used for measurement in a mass spectrometer in [3-1 Measurement of calibrants] that will be described later.

### [Example 2: Production of calibrant on weight basis]

Fig. 4 is a schematic diagram showing a production method of a calibrant according to Example 2. As Example 2, five kinds of calibrants IC-1, IC-2, IC-3, IC-4, and IC-5 were produced which contain Aβ1-38 (SEQ ID NO.: 11) and a stable isotope-labeled Aβ1-38 (SIL-Aβ1-38) as calibration substances and are respectively different in a ratio (a concentration ratio Aβ1-38/SIL-Aβ1-38) of a concentration of Aβ1-38 to a concentration of SIL-Aβ1-38, as in the case of Example 1. The concentration of SIL-Aβ1-38 in each of the five kinds of calibrants of Example 2 was the same as that in each of the respective calibrants of Example 1. The concentration of Aβ1-38 in each of the five kinds of calibrants of Example 2 was the same as that in each of the respective calibrants of Example 1.

The calibrants in Example 2 was produced as follows.

### [2-1 Preparation of solution A and solution B]

In the same manner as in Example 1, SIL-Aβ1-38 was dissolved in a solvent (20% acetonitrile aqueous solution containing 1 mg/mL BSA and 50 mM ammonium bicarbonate) to prepare a solution A0 (6-peptide-A solution). The concentration of SIL-Aβ1-38 in the solution A0 (6-peptide-A solution) was 100 nmol/L as in the case of Example 1. The solution A0 further contained Aβ1-33 (300 nmol/L), Aβ1-34 (200 nmol/L), Aβ1-36 (100 nmol/L), Aβ1-37 (50 nmol/L), and APP669-711 (50 nmol/L) as in Example 1.

Further, 220 µL of the prepared solution A0 (6-peptide-A solution) was mixed with 8140 µL of a solvent for IC concentrated solutions (20% acetonitrile aqueous solution containing 0.1 mg/mL BSA and 50 mM ammonium bicarbonate) to prepare a solution A0' (6-peptide-A2 solution). The solution A0' was a thirty-eight-fold diluted solution of the solution A0. The concentration of SIL-Aβ1-38 in the solution A0' (6-peptide-A2 solution) was approximately 2.63 nmol/L.

Aβ1-38 was dissolved in the solvent (20% acetonitrile aqueous solution containing 1 mg/mL BSA and 50 mM ammonium bicarbonate) to prepare a solution B0 (Aβ1-38 solution). The concentration of Aβ1-38 in the solution B0 (Aβ1-38 solution) was 800 nM (nmol/L) as in the case of Example 1.

3800 µL of the solution A0' (6-peptide-A2 solution) and 200 µL of the solvent for IC concentrated solutions (20% acetonitrile aqueous solution containing 0.1 mg/mL BSA and 50 mM ammonium bicarbonate) were mixed to prepare a solution A (6-peptide-B). The concentration of SIL-Aβ1-38 in the solution A (6-peptide-B) was 2.5 nmol/L as in the case of Example 1. The solution A resulted in being a forty-fold diluted solution of the solution A0. The concentration of SIL-Aβ1-38 in the solution A (6-peptide-B) was adjusted to a desired SIL-Aβ1-38 concentration in the five calibrants IC-1, IC-2, IC-3, IC-4, and IC-5 to be produced.

3800 µL of the solution A0' (6-peptide-A2 solution), 100 µL of the solvent for IC concentrated solutions (20% acetonitrile aqueous solution containing 0.1 mg/mL BSA and 50 mM ammonium bicarbonate), and 100 µL of the solution B0 (Aβ1-38 solution) were mixed to prepare a solution B (IC-0). The concentration of SIL-Aβ1-38 in the solution B (IC-0) was, as in the case of Example 1, 2.5 nmol/L that is equal to the concentration of SIL-Aβ1-38 in the solution A (6-peptide-B). The concentration of Aβ1-38 in the solution B (IC-0) was 20 nmol/L as in the case of Example 1.

As described above, in Example 2, the solution A was prepared by adding, to 3800 µL of the solution A0', a clearly smaller amount of the solvent. The solution B was prepared by adding, to 3800 µL of the solution A0', clearly smaller amounts of the solvent and the solution B0. It is considered that the concentration of SIL-Aβ1-38 in each of the solution A and the solution B in Example 2 could be more accurately a certain predetermined value than the concentration of SIL-Aβ1-38 in each of the solution A and the solution B in Example 1.

All measuring in 2-1 of Example 2 was done by volume by pipetting. A pipette of Eppendorf Research Plus was used.

### [2-2 Preparation of calibrants]

Next, the prepared solution B was sequentially diluted with the solution A to produce IC-1, IC-2, IC-3, IC-4, and IC-5 in the following manner.

750 mg of the prepared solution A and 750 mg of the prepared solution B were mixed to produce IC-1 solution. The ratio (the concentration ratio Aβ1-38/SIL-Aβ1-38) of the concentration of Aβ1-38 to the concentration of SIL-Aβ1-38 in the IC-1 solution was 4. 750 mg of the produced IC-1 solution and 750 mg of the solution A were mixed to produce IC-2 solution. The concentration ratio Aβ1-38/SIL-Aβ1-38 in the IC-2 solution was 2. 750 mg of the produced IC-2 solution and 750 mg of the solution A were mixed to produce IC-3 solution. The concentration ratio Aβ1-38/SIL-Aβ1-38 in the IC-3 solution was 1. 750 mg of the produced IC-3 solution and 750 mg of the solution A were mixed to produce IC-4 solution. The concentration ratio Aβ1-38/SIL-Aβ1-38 in the IC-4 solution was 1/2. 750 mg of the produced IC-4 solution and 750 mg of the solution A were mixed to produce IC-5 solution. The concentration ratio Aβ1-38/SIL-Aβ1-38 in the IC-5 solution was 1/4.

All measuring in 1-2 of Example 2 was done by weight by an electronic balance. As the electronic balance, an AUX-220 manufactured by Shimadzu Corporation was used. The repeatability error of this electronic balance was 0.1 mg as a standard deviation. The weighing error in the electronic balance is considered to be very small.

Each of two operators produced calibrants IC-1, IC-2, IC-3, IC-4, and IC-5 once following the above procedure. The produced calibrants were preserved by freezing. Each of the produced calibrants was diluted ten-fold and used for measurement in a mass spectrometer in [3-1 Measurement of calibrants] that will be described later.

### [Example 3: Measurement of calibrants and calculation of calibration value]

### [3-1 Measurement of calibrants]

Table 1 shows the five kinds of liquid concentrate (IC-1 to IC-5) of intensity ratio calibrant (IC) reagents, which were produced in Example 1 and Example 2. Before MS measurement, IC-1 to IC-5 were thawed and diluted 10-fold with a IP eluent (5 mM HCl, 0.1 mM Methionine, 70% (v/v) acetonitrile) that can be used in immunoprecipitation to prepare diluted solutions (IC-1 diluted solution to IC-5 diluted solution) of IC-1 to IC-5, respectively. In advance, 0.5 µL of 0.5 mg/mL CHCA/0.2% (w/v) MDPNA was dropped into each well of a µFocus MALDI plate^{™} 900µm and dried. Each of the IC-1 to IC-5 was dropped into four wells in an amount of 1 µL per well and dried. The protein/peptide compositions of the IC-1 diluted solution to IC-5 diluted solution are shown in Table 2. The abundance ratios of Aβ1-38 to SIL-Aβ1-38 in the IC-1 to IC-5 were set to 4, 2, 1, 1/2, and 1/4, respectively. The abundance ratios of Aβ1-38 to SIL-Aβ1-38 are not affected by dilution. The abundance ratios of Aβ1-38 to SIL-Aβ1-38 in the IC-1 diluted solution to the IC-5 diluted solution were also 4, 2, 1, 1/2, and 1/4, respectively.

**[Table 1]**

| | IC-1 | IC-2 | IC-3 | IC-4 | IC-5 |
|---|---|---|---|---|---|
| Aβ1-38 | 10 fmol/µL | 5 fmol/µL | 2.5 fmol/µL | 1.25 fmol/µL | 0.625 fmol/µL |
| SIL-Aβ1-38 | 2.5 fmol/µL | | | | |
| Others | Aβ1-33 (7.5 fmol/µL), Aβ1-34 (5 fmol/µL), Aβ1-36 (2.5 fmol/µL), Aβ1-37 (1.25 fmol/µL), APP669-711 (1.25 fmol/µL), BSA (100 ng/µL) | | | | |

**[Table 2]**

| | IC-1 diluted solution | IC-2 diluted solution | IC-3 diluted solution | IC-4 diluted solution | IC-5 diluted solution |
|---|---|---|---|---|---|
| Aβ1-38 | 1000 amol/µL | 500 amol/µL | 250 amol/µL | 125 amol/µL | 62_{.}5 amol/µL |
| SIL-Aβ1-38 | 250 amol/µL | | | | |
| Others | Aβ1-33 (750 amol/µL), Aβ1-34 (500 amol/µL), Aβ1-36 (250 amol/µL), Aβ1-37 (125 amol/µL), APP669-711 (125 amol/µL), BSA (10 ng/µL) | | | | |

Mass spectrum data were obtained using AXIMA Performance (Shimadzu/KRATOS, Manchester, UK) by Linear TOF in a positive ion mode. The m/z value of Linear TOF was indicated by the average mass of peaks. The m/z value was calibrated using, as external standards, human angiotensin II, human ACTH fragment 18-39, bovine insulin oxidized beta-chain, and bovine insulin. A mass spectrum was obtained by integrating 40 shots per each of the points of 400 spots in a raster mode. As a quantitative value, an average of peak intensity ratios of Aβ1-38 relative to SIL-Aβ1-38 in spectra obtained by measuring the 4 wells was used. A detection limit was an S/N ratio of 3, and peaks of not more than the detection limit were regarded as not detectable.

The IC-1 diluted solution to the IC-5 diluted solution were subjected to the above-described measurement.

Power approximate equations were prepared from the intensity ratios of Aβ1-38 relative to SIL-Aβ1-38 in the mass spectra of the IC-1 diluted solution to the IC-5 diluted solution and the concentration ratios (the abundance ratios) of Aβ1-38 relative to SIL-Aβ1-38 in the IC-1 diluted solution to the IC-5 diluted solution.

Power approximate equation: y = ax^{b},
wherein x is the abundance ratio of Aβ1-38/SIL-Aβ1-38, y is the peak intensity ratio of Aβ1-38/SIL-Aβ1-38, and a and b are coefficients in the approximate equation.

The value a in the power approximate equation does not change depending on machine conditions, but the value b is greatly influenced by machine conditions. Therefore, calibration using only the value b makes it possible to calibrate a difference between mass spectrometer machines accurately.

When the value b is adjusted to fall within a certain range, the signal peak intensity ratios of the analyte substances can more accurately be calibrated. For example, the value b may be adjusted in a range of 0.9 to 1.1.

Table 3 shows the results when the calibrants of Example 1 were used. The above-described measurement for the IC-1 diluted solution to the IC-5 diluted solution was performed for each set of the calibrants that were produced three times by each of the three operators to calculate the value b.

**[Table 3]**

| | **IC3 int.ratio** | **IC1 int.ratio** | **a** | **b** | **Average** | **STDEV.P** | **CV(%)** |
|---|---|---|---|---|---|---|---|
| **N1** | 0.9177 | 4.2022 | 0.9373 | **1.0713** | 1.0696 | 0.0093 | 0.87 |
| **N2** | 0.8718 | 4.0495 | 0.9086 | **1.0575** | | | |
| **N3** | 0.9653 | 4.4320 | 0.9739 | **1.0801** | | | |
| **Y1** | 0.9238 | 4.0673 | 0.9380 | **1.0512** | 1.0527 | 0.0041 | 0.39 |
| **Y2** | 1.0534 | 4.5455 | 1.0532 | **1.0584** | | | |
| **Y3** | 0.9892 | 4.3641 | 1.0062 | **1.0487** | | | |
| **F1** | 1.2052 | 4.8896 | 1.2069 | **1.0150** | 1.0231 | 0.0179 | 1.75 |
| **F2** | 0.6818 | 3.0113 | 0.7200 | **1.0063** | | | |
| **F3** | 0.9851 | 4.1910 | 0.9784 | **1.0478** | | | |

"IC3 int.ratio" in Table 3 indicates the peak intensity ratio of Aβ1-38 relative to SIL-Aβ1-38 in the IC-3 diluted solution. "IC1 int.ratio" indicates the peak intensity ratio of Aβ1 -38 relative to SIL-Aβ1-38 in the IC-1 diluted solution. "a" and "b" indicate coefficients in the above power approximate equation (y = ax^{b}). "Average" indicates an average value of the values b for the calibrants produced three times by the same operator. "STDEV.P" indicates a standard deviation of the values b for the calibrants produced three times by the same operator. "CV (%)" indicates a coefficient of variation of the values b for the calibrants produced three times by the same operator.

When the value b was calculated using the calibrants of Example 1, the values b were in a range of 1.0063 to 1.0801. In Example 1, the values b in all cases were within a range of 0.9 to 1.1.

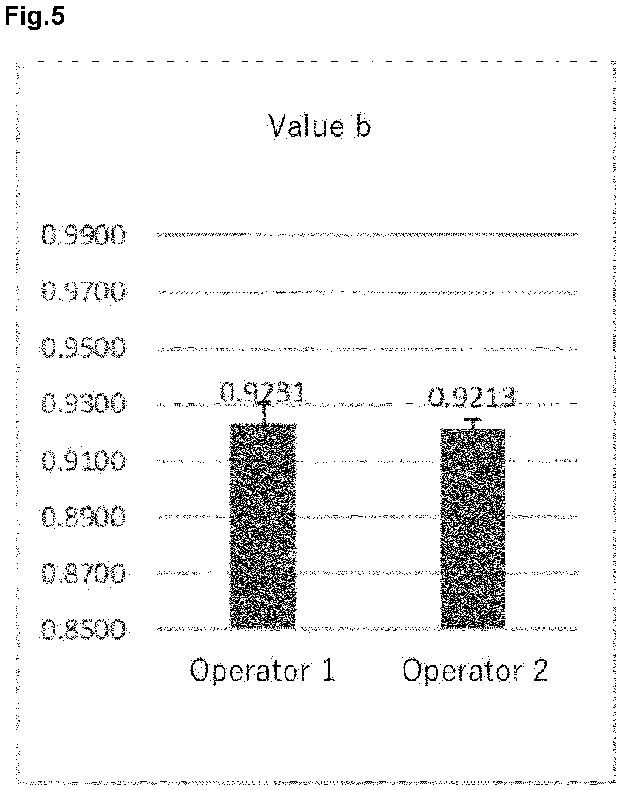

Fig. 5 shows the results when the calibrants of Example 2 were used. The above-described measurement for the IC-1 diluted solution to the IC-5 diluted solution was performed three times in total for each set of the calibrants that were produced once by each of the operator 1 and the operator 2 to calculate the value b in each of the measurement. Fig. 5 is a bar chart showing an average value of the values b calculated by using the calibrants produced by each operator. The vertical axis represents the value b. The error bar represents a standard deviation of three values b obtained from three measurement of the calibrants produced by each operator.

When the value b was calculated using the calibrants of Example 2, the average values of the values b calculated by using the calibrants produced by two operators were 0.9231 and 0.9213, respectively, and roughly equivalent value b were obtained irrespective of the operators. Thus, the variation in the values b calculated by using the calibrants of Example 2 was further reduced than that in the values b calculated by using the calibrants of Example 1.

One reason for the smaller variation in the values b in Example 2 may be considered that the ratio (the concentration ratio Aβ1-38/SIL-Aβ1-38) of the concentration of Aβ1-38 to the concentration of SIL-Aβ1-38 was more accurately adjusted in each of the calibrants in Example 2.

In Example 2, when the solution B was sequentially diluted with the solution A, measuring was done by weight using the electronic balance. The solvent used for the calibrants in Example 1 and Example 2 contains BSA and acetonitrile, and thereby being viscos and affecting pipetting due to volatilization of acetonitrile. When such a solution is measured, weight measuring by using the electronic balance is considered to reduce measuring error compared to pipetting. Further, measuring by the electronic balance is considered to have smaller differences due to repetition and smaller differences among operators than pipetting.

Therefore, it is considered that, in Example 2, the concentration of Aβ1-38 in each of IC-1, IC-2, IC-3, IC-4 and IC-5 was prepared to each predetermined concentration more accurately than in Example 1. That is, it is considered that the concentration ratio Aβ1-38/SIL-Aβ1-38 in each of IC-1, IC-2, IC-3, IC-4 and IC-5 was prepared more accurately. Further, it is considered that variation among operators was small, and therefore the calibrant prepared to the accurate concentration ratio Aβ1-38/SIL-Aβ1-38 could be produced with high reproducibility.

In Example 2, the solution A0 was diluted 38-fold to prepare the solution A0'. Then, the solution A and the solution B were respectively prepared by using the same amount of the prepared solution A0'. Thus, it is considered that, in Example 2, the concentration of SIL-Aβ1-38 in each of IC-1, IC-2, IC-3, IC-4 and IC-5 was prepared to each predetermined concentration more accurately than in Example 1. That is, it is considered that the concentration ratio Aβ1-38/SIL-Aβ1-38 in each of IC-1, IC-2, IC-3, IC-4 and IC-5 was prepared more accurately.

The present invention includes, for example, the following aspects.
(1) A calibrant for use in a mass spectrometer, the calibrant comprising not less than two calibration substances, wherein
   a ratio of, relative to a concentration of one calibration substance of the not less than two calibration substances, a concentration of another calibration substance of the not less than two calibration substances is a predetermined value.
(2) The calibrant according to the above (1), wherein the calibration substances include a substance labeled with a stable isotope and a substance not labeled with a stable isotope.
(3) The calibrant according to the above (1) or (2), wherein the calibration substances are Aβ related peptides.
(4) The calibrant according to any one of the above (1) to (3), wherein the calibration substances are Aβ1-38 and a stable isotope-labeled Aβ1-38.
(5) The calibrant according to any one of the above (1) to (4), wherein the calibrant comprises a plurality of calibrants which are respectively different in a concentration of at least one calibration substance of the not less than two calibration substances.
(6) The calibrant according to any one of the above (1) to (5), wherein a ratio of, relative to a concentration of one calibration substance of the not less than two calibration substances, a concentration of another calibration substance of the not less than two calibration substances is in a range of 1/4 to 4.
(7) A method for producing a calibrant for use in a mass spectrometer, the calibrant comprising not less than two calibration substances, wherein
   a ratio of, relative to a concentration of one calibration substance of the not less than two calibration substances, a concentration of another calibration substance of the not less than two calibration substances is a predetermined value, and
   the method comprising:
      a previous step of preparing:
      a solution A which comprises one calibration substance (S 1) of the not less than two calibration substances at a predetermined concentration, and
      a solution B which comprises the one calibration substance (S1) of the not less than two calibration substances at a concentration equal to the above predetermined concentration, and further comprises another calibration substance (S2) of the not less than two calibration substances; and
      a preparation step of obtaining a calibrant in which a ratio of, relative to a concentration of the one calibration substance (S1), a concentration of the another calibration substance (S2) is a predetermined value by using the solution A and the solution B.
(8) The method according to the above (7), wherein, in the preparation step, a plurality of calibrants which are constant in a concentration of the one calibration substance (S1) and respectively different at least in a concentration of the another calibration substance (S2) are obtained.
(9) The method according to the above (7), wherein, in the preparation step, the calibrant in which the ratio of, relative to the concentration of the one calibration substance (S 1), the concentration of the another calibration substance (S2) is the predetermined value is obtained by using the solution A and the solution B,
   a mixing operation is performed to separate a part of the calibrant from the resulting calibrant and further mix the part of the calibrant separated with the solution A, and thereby
   another calibrant in which a ratio of, relative to a concentration of the one calibration substance (S1), a concentration of the another calibration substance (S2) is a different value from the predetermined value is obtained.
(10) The method according to the above (7), wherein, in the preparation step, the calibrant in which the ratio of, relative to the concentration of the one calibration substance (S 1), the concentration of the another calibration substance (S2) is the predetermined value is obtained by using the solution A and the solution B,
   a mixing operation is performed to separate a part (for example, a half) of the calibrant from the resulting calibrant and further mix the part of the calibrant separated with the solution A in an amount equal to the part of the calibrant separated, and thereby
   another calibrant in which a ratio of, relative to a concentration of the one calibration substance (S1), a concentration of the another calibration substance (S2) is 1/2 of the predetermined value is obtained.
(11) The method according to the above (9) or (10), wherein, in the preparation step, the mixing operation is performed two or more times sequentially.
(12) The method according to the above (7), wherein, in the preparation step, the calibrant in which the ratio of, relative to the concentration of the one calibration substance (S1), the concentration of the another calibration substance (S2) is the predetermined value is obtained by using the solution A and the solution B,
   a mixing operation is performed to separate a part of the calibrant from the resulting calibrant and further mix the part of the calibrant separated with the solution B, and thereby
   another calibrant in which a ratio of, relative to a concentration of the one calibration substance (S1), a concentration of the another calibration substance (S2) is a different value from the predetermined value is obtained.
(13) The method according to the above (7), wherein, in the preparation step, the calibrant in which the ratio of, relative to the concentration of the one calibration substance (S1), the concentration of the another calibration substance (S2) is the predetermined value is obtained by using the solution A and the solution B,
   a mixing operation is performed to separate a part (for example, a half) of the calibrant from the resulting calibrant and further mix the part of the calibrant separated with the solution B in an amount equal to the part of the calibrant separated, and thereby
   another calibrant in which a ratio of, relative to a concentration of the one calibration substance (S1), a concentration of the another calibration substance (S2) is a different value from the predetermined value is obtained.
(14) The method according to the above (12) or (13), wherein, in the preparation step, the mixing operation is performed two or more times sequentially.
(15) The method according to any one of the above (7) to (14), wherein the preparation step using the solution A and the solution B is performed by volume measuring.
(16) The method according to any one of the above (7) to (14), wherein the preparation step using the solution A and the solution B is performed by weight measuring.
(17) The method according to any one of the above (7) to (16), wherein
   the one calibration substance (S1) is a substance labeled with a stable isotope, and
   the another one calibration substance (S2) is a substance not labeled with a stable isotope.
(18) The method according to any one of the above (7) to (17), wherein
   the one calibration substance (S1) is Aβ1-38 labeled with a stable isotope, and
   the another one calibration substance (S2) is Aβ1-38 not labeled with a stable isotope.
(19) A calibrant kit for use in a mass spectrometer, the kit comprising a plurality of calibrants which comprises not less than two calibration substances, wherein
   the plurality of calibrants are respectively different in a concentration of at least one calibration substance of the calibration substances.
(20) A calibrant kit for use in a mass spectrometer, the kit comprising not less than two calibration substances.
(21) The calibrant kit according to the above (20), wherein the kit further comprises a solvent.
(22) The calibrant kit according to the above (20) or (21), wherein the kit further comprises a container to accommodate a calibrant.

## Claims

1. A calibrant for use in a mass spectrometer, the calibrant comprising not less than two calibration substances, wherein
a ratio of, relative to a concentration of one calibration substance of the not less than two calibration substances, a concentration of another calibration substance of the not less than two calibration substances is a predetermined value.

2. The calibrant according to claim 1, wherein the calibration substances include a substance labeled with a stable isotope and a substance not labeled with a stable isotope.

3. The calibrant according to claim 1, wherein the calibration substances are Aβ related peptides.

4. The calibrant according to claim 1, wherein the calibration substances are Aβ1-38 and a stable isotope-labeled Aβ1-38.

5. The calibrant according to claim 1, wherein the calibrant comprises a plurality of calibrants which are respectively different in a concentration of at least one calibration substance of the not less than two calibration substances.

6. The calibrant according to claim 1, wherein a ratio of, relative to a concentration of one calibration substance of the not less than two calibration substances, a concentration of another calibration substance of the not less than two calibration substances is in a range of 1/4 to 4.

7. A method for producing a calibrant for use in a mass spectrometer, the calibrant comprising not less than two calibration substances, wherein
a ratio of, relative to a concentration of one calibration substance of the not less than two calibration substances, a concentration of another calibration substance of the not less than two calibration substances is a predetermined value, and
the method comprising:
a previous step of preparing:
a solution A which comprises one calibration substance (S1) of the not less than two calibration substances at a predetermined concentration, and
a solution B which comprises the one calibration substance (S1) of the not less than two calibration substances at a concentration equal to the predetermined concentration, and further comprises another calibration substance (S2) of the not less than two calibration substances; and
a preparation step of obtaining a calibrant in which a ratio of, relative to a concentration of the one calibration substance (S1), a concentration of the another calibration substance (S2) is a predetermined value by using the solution A and the solution B.

8. The method according to claim 7, wherein, in the preparation step, a plurality of calibrants which are constant in a concentration of the one calibration substance (S1) and respectively different at least in a concentration of the another calibration substance (S2) are obtained.

9. The method according to claim 7, wherein, in the preparation step, the calibrant in which the ratio of, relative to the concentration of the one calibration substance (S1), the concentration of the another calibration substance (S2) is the predetermined value is obtained by using the solution A and the solution B,
a mixing operation is performed to separate a part of the calibrant from the resulting calibrant and further mix the part of the calibrant separated with the solution A, and thereby
another calibrant in which a ratio of, relative to a concentration of the one calibration substance (S1), a concentration of the another calibration substance (S2) is a different value from the predetermined value is obtained.

10. The method according to claim 7, wherein, in the preparation step, the calibrant in which the ratio of, relative to the concentration of the one calibration substance (S1), the concentration of the another calibration substance (S2) is the predetermined value is obtained by using the solution A and the solution B,
a mixing operation is performed to separate a part of the calibrant from the resulting calibrant and further mix the part of the calibrant separated with the solution A in an amount equal to the part of the calibrant separated, and thereby
another calibrant in which a ratio of, relative to a concentration of the one calibration substance (S1), a concentration of the another calibration substance (S2) is 1/2 of the predetermined value is obtained.

11. The method according to claim 9, wherein, in the preparation step, the mixing operation is performed two or more times sequentially.

12. The method according to claim 7, wherein, in the preparation step, the calibrant in which the ratio of, relative to the concentration of the one calibration substance (S1), the concentration of the another calibration substance (S2) is the predetermined value is obtained by using the solution A and the solution B,
a mixing operation is performed to separate a part of the calibrant from the resulting calibrant and further mix the part of the calibrant separated with the solution B, and thereby
another calibrant in which a ratio of, relative to a concentration of the one calibration substance (S1), a concentration of the another calibration substance (S2) is a different value from the predetermined value is obtained.

13. The method according to claim 7, wherein, in the preparation step, the calibrant in which the ratio of, relative to the concentration of the one calibration substance (S1), the concentration of the another calibration substance (S2) is the predetermined value is obtained by using the solution A and the solution B,
a mixing operation is performed to separate a part of the calibrant from the resulting calibrant and further mix the part of the calibrant separated with the solution B in an amount equal to the part of the calibrant separated, and thereby
another calibrant in which a ratio of, relative to a concentration of the one calibration substance (S1), a concentration of the another calibration substance (S2) is a different value from the predetermined value is obtained.

14. The method according to claim 12, wherein, in the preparation step, the mixing operation is performed two or more times sequentially.

15. The method according to claim 7, wherein the preparation step using the solution A and the solution B is performed by volume measuring.

16. The method according to claim 7, wherein the preparation step using the solution A and the solution B is performed by weight measuring.

17. The method according to claim 7, wherein
the one calibration substance (S1) is a substance labeled with a stable isotope, and
the another one calibration substance (S2) is a substance not labeled with a stable isotope.

18. The method according to claim 7, wherein
the one calibration substance (S1) is Aβ1-38 labeled with a stable isotope, and
the another one calibration substance (S2) is Aβ1-38 not labeled with a stable isotope.

19. A calibrant kit for use in a mass spectrometer, the kit comprising a plurality of calibrants which comprises not less than two calibration substances, wherein the plurality of calibrants are respectively different in a concentration of at least one calibration substance of the calibration substances.

20. A calibrant kit for use in a mass spectrometer, the kit comprising not less than two calibration substances.

21. The calibrant kit according to claim 20, wherein the kit further comprises a solvent.

22. The calibrant kit according to claim 20, wherein the kit further comprises a container to accommodate a calibrant.
